(19) Europäisches Patentamt
European Patent Office
Office européen des brevets

(11) EP 0 552 516 B1

(12) **EUROPÄISCHE PATENTSCHRIFT**

(45) Veröffentlichungstag und Bekanntmachung des Hinweises auf die Patenterteilung:
**10.09.1997 Patentblatt 1997/37**

(51) Int. Cl.$^6$: **A61K 35/50**, A61K 35/55

(21) Anmeldenummer: **92250349.5**

(22) Anmeldetag: **02.12.1992**

(54) **Wässrige synthetische Organextrakte**

Aqueous synthetic organ-extracts

Extraits aqueux synthétiques d'organes

(84) Benannte Vertragsstaaten:
**AT BE CH DE DK ES FR GB IT LI NL SE**

(30) Priorität: **02.12.1991 DE 4139639**
**18.08.1992 DE 4227633**

(43) Veröffentlichungstag der Anmeldung:
**28.07.1993 Patentblatt 1993/30**

(73) Patentinhaber:
**Riemschneider, Randolph, Dr. Dr. Prof.**
**14052 Berlin (DE)**

(72) Erfinder: **Der Erfinder hat auf seine Nennung verzichtet.**

(74) Vertreter: **Hartmann, Günter, Dr. Dipl.-Chem.**
**Ruschke Hartmann Becker**
**Pienzenauerstrasse 2**
**81679 München (DE)**

(56) Entgegenhaltungen:
**DE-A- 2 338 970**          **DE-A- 2 405 983**

Printed by Rank Xerox (UK) Business Services
2.14.14/3.4

**Beschreibung**

Die Erfindung betrifft wäßrige synthetische Organextrakte, Verfahren zu deren Herstellung sowie pharmazeutische und kosmetische Zusammensetzungen, die synthetische Organextrakte beinhalten.

Organextrakte besitzen als Grundstoffe für Pharmaka und Kosmetika eine große Bedeutung. Fraktionierte Milz-, Blut-, Placenta- und Thymusextrakte zum Beispiel werden als Wirkstoffe in Pharmaka und Kosmetika eingesetzt (Seifen - Öle - Fette - Wachse 112 (1986) 215-218), wobei sie in vielen Fällen durch niedermolekulare Zusätze ergänzt werden.

Organ-Extrakte zeichnen sich durch eine Vielzahl von Inhalts- bzw. Wirkstoffen aus, die entweder als vollständiger Extrakt oder in Form daraus isolierter Einzelsubstanzen Verwendung finden. Für Placenta-Extrakte wurden beispielsweise über 100 Komponenten nachgewiesen (vgl. *Kosmetik International* 6 (1978) 1-8). Für einen Teil bestimmter Einzelsubstanzen der Organextrakte ist die Synthese bisher nicht gelungen, zumal der dazu nötige Aufwand oft zu groß ist.

Zahlreiche Organextrakte stehen heute in mehr oder weniger naturbelassener Form zur Verfügung, wie z.B. Blutserumextrakte, Placentaextrakte, Thymusextrakte, Kollagenextrakte und Bindegewebsextrakte. Solche Präparate enthalten vielfach noch Proteine oder Protein-Abbauprodukte, die immunogene bzw. pathogene Eigenschaften aufweisen und bei regelmäßiger oder wiederholter Applikation - meistens verbunden mit sehr langen Inkubationszeiten - zu schwerwiegenden Erkrankungen führen können. Dabei wird insbesondere auf die durch unkonventionelle Viren und Prionen wie z.B BSE verursachten Krankheitsbilder Bezug genommen. Es sind auch proteinhaltige Placentaextrakte bekannt, die sich bei der Anwendung für den Menschen als krebserregend erwiesen haben (R. Riemschneider, G. Quelle, "Carcinogenity of Placenta Extracts?", Fragrance Jounal Bd. 57, 10, Nr. 4, 115-122 (1982) und Bd. 57, 10, Nr. 6. 105-112 (1982)).

In der DE-PS 2 338 970 wird ein Verfahren zur Herstellung eines Placentaextraktes beschrieben, welcher frei von Lipoidbegleitstoffen und frei von Proteinen ist. Bei der Anwendung dieses Extraktes wurde jedoch eine erhebliche Beeinträchtigung des mit dem Naturstoff ursprünglich erzielten Wirkungsspektrums beobachtet.

Der "synthetische" Weg einer Kombination niedermolekularer Komponenten in Anlehnung an die in entsprechenden Naturstoffextrakten gefundenen Zusammensetzungen ist ebenfalls beschritten worden. So wird z.B. in der DE-AS 2 405 983 ein Verfahren zur Herstellung eines atmungsfördernden Präparates beschrieben, welches im wesentlichen aus niedermolekularen Komponenten sowie aus Zusätzen tierischen Ursprungs zusammengesetzt ist.

In der DE-OS 2 021 969 wird die Herstellung eiweißfreier Präparate durch Kombination verschiedener in Blutextrakten nachweisbarer Komponenten beschrieben. Obwohl hiermit das Proteinproblem behoben werden konnte, ist mit den bisherigen Vorschlägen nur eine unzureichende Annäherung an einzelne Eigenschaften von natürlichen Organextrakten gelungen. Dies mag bei bestimmten Anwendungen wie im Bereich des Futtermittel- und Nährstoffsektors hinnehmbar sein. Insbesondere bei kosmetischen und medizinischen Anwendungen wird aber gerade ein wirksames physiologisches Aktivitätsprofil gewünscht, das mit den bisher bekannten Zusammensetzungen synthetischer oder halbsynthetischer Natur nicht oder nur unzureichend geschaffen werden konnte. Ein allgemeines und standardisierbares Konzept zum Aufbau synthetischer Organextrakte fehlte bisher.

Der Erfindung liegt die Aufgabe zu Grunde, synthetische Organextrakte zu schaffen, die das biochemische Wirkungsprofil von natürlichen Organextrakten mindestens erreichen, vorzugsweise jedoch noch wesentlich verbessern, andererseits aber frei sind von gegebenenfalls vorhandenen immunogenen bzw. pathogenen Proteinen und Protein-Abbauprodukten. Diese synthetischen Organextrakte sollten wesentlich einfacher und reproduzierbar herzustellen sowie in gegebenenfalls pyrogenfreier Form zugänglich sein. Insbesondere gilt dies für die Verwendung der erfindungsgemäßen synthetischen Organextrakte zur Herstellung von kosmetischen und medizinischen Formulierungen. Desweiteren soll erfindungsgemäß die Verwendung flüchtiger, meist brennbarer und oft toxischer Lösungsmittel zur Herstellung von Organextrakten vermieden werden.

Zur Lösung der Aufgabe werden die erfindungsgemäßen synthetischen Organextrakte gemäß Hauptanspruch vorgeschlagen, die mindestens

(a) eine Aminosäurekomponente mit monomeren Aminosäuren oder Aminosäurederivaten,
(b) eine Peptidkomponente,
(c) eine Nucleobase, ein Nucleosid-, Nucleotid- oder Nucleinsäurekomponente,
(d) eine Kohlenhydratkomponente und/oder eine Kohlenhydratderivatkomponente,
(e) eine aliphatische Carbonsäure oder deren Salz mit 3 bis 6 Kohlenstoffatomen,
(f) einen aliphatischen und/oder aromatischen Alkohol mit 2 bis 7 Kohlenstoffatomen,
        sowie gegebenenfalls
(g) Vitamine,
(h) Mineralsalze und/oder Spurenelemente,
(i) Puffersubstanzen und
(k) Konservierungsstoffe

enthält und **dadurch gekennzeichnet** ist, daß die Aminosäurekomponente (a) und die Peptidkomponente (b) eine oder mehrere der zu den Gruppen

(I) Glycin, L-Prolin, L-Hydroxyprolin, L-Alanin,

(II) L-Glutaminsäure, L-Asparaginsäure, L-Asparagin,

(III) L-Arginin, L-Serin, L-Lysin

gehörenden Aminosäuren enthält, wobei sich der Extrakt zu mindestens

- 0,20 Gew.% aus Aminosäure(n) der Gruppe (I),
- 0,05 Gew.% aus Aminosäure(n) der Gruppe (II) und
- 0,05 Gew.% aus Aminosäure(n) der Gruppe (III)

zusammensetzt, und

- die Komponente (c) mit einem Anteil von mindestens 0,02 Gew.%,
- die Komponente (d), die mindestens einen Zuckeralkohol aus der Gruppe bestehend aus Sorbit, Mannit, Inosit und Dulcit umfaßt, mit einem Anteil von mindestens 0,2 Gew.%,
- die Komponente (e) mit einem Anteil von mindestens 0,2 Gew.%, und
- die Komponente (f) mit einem Anteil von mindestens 0,3 Gew.%

jeweils bezogen auf den Gesamtextrakt, in der synthetischen Extraktlösung enthalten ist.

Es wurde überraschenderweise gefunden, daß die vorstehenden Auswahlkriterien für die Wirksamkeit eines synthetischen Organextraktes wesentlich sind. Mit einer auf diesen Kriterien aufbauenden Zusammensetzung wurde erfindungsgemäß ein generalisierbares Konzept für den Aufbau eines synthetischen Organextraktes gefunden, das je nach Anwendungsrichtung nur geringfügig modifiziert zu werden braucht, um das vollständige Wirkungsprofil des jeweiligen Organextraktes natürlichen Ursprungs zu erreichen. Auf der Basis der erfindungsgemäßen Lehre liegt diese Modifizierung im Rahmen des Könnens des durchschnittlichen Fachmannes auf dem einschlägigen Gebiet.

Im Gegensatz zu natürlichen Organextrakten garantieren die erfindungsgemäßen wäßrigen synthetischen Organextrakte einen gleichbleibenden, d.h. reproduzierbaren Qualitätsstandard und damit ein gleichbleibendes Wirkungsprofil. Die zellatmungsaktivierenden Eigenschaften der erfindungsgemäßen wäßrigen synthetischen Organextrakte übersteigen diejenigen herkömmlicher Organextrakte.

Das jeweilige Wirkungsspektrum eines entsprechenden Naturstoffextraktes läßt sich mit diesem Konzept erreichen bzw. verbessern, und auf die Anwesenheit von pathogenen bzw. virulogen Proteinen wird verzichtet, sowie auf Protein-Abbauprodukte und Hormone zurückzuführende Beeinträchtigungen können vermieden werden.

Die erfindungsgemäßen wäßrigen synthetischen Organextrakte zeichnen sich insbesondere durch eine Erhöhung der Zellproliferation und eine positive Beeinflussung des Keratinisierungsprozesses aus. Eine noch weiter beschleunigte Wundheilung, verbunden mit einer Förderung der Granulation sowie eine Stimulierung der Epithelisierung sind neben einer Verbesserung des Feuchthaltevermögens der Haut (Moisturising effect), einer Normalisierung des Fettgehaltes sowie einer besseren Schutzwirkung vor Radikalen, besonders in hydrophilen inter- und intrazellulären Bereichen, als besondere Vorteile der erfindungsgemäßen synthetischen wäßrigen Organextrakte zu nennen. Die Formulierungen führen zur Durchblutungsförderung und zur allgemeinen Linderung von Hautreizungen. Es kommt somit nicht nur zur Verminderung des Erythems und zur Verbesserung der Hautregeneration beispielsweise nach Sonnenbrand, sondern es wird eine noch weitere, allgemeine Verbesserung des Hautzustandes, wie zum Beispiel Hautglättung und Geschmeidigkeit der Haut, erreicht.

Ferner bezieht sich die Erfindung auf ein vorteilhaftes Verfahren zur Herstellung dieser synthetischen Organextrakte sowie auf deren Verwendung zur Herstellung von kosmetischen und medizinischen Formulierungen, die eine wirksame Menge dieser synthetischen Organextrakte enthalten. Die medizinischen Präparate mit solchen Wirkstoffen sind insbesondere zur topischen, subkutanen und/oder intramuskulären Applikation für die Zwecke der äußeren und inneren Wundheilung, zur Stärkung des Immunsystems bzw. zur Aktivierung des Zellstoffwechsels geeignet. Gegenüber bisher bekannten Organextrakten können mit den erfindungsgemäßen wäßrigen synthetischen Organextrakten sowohl Zellstoffwechsel als auch Wundheilung in noch vorteilhafterer Weise verbessert werden. Ferner werden die erfindungsgemäßen Organextrakte bevorzugt zur Herstellung gastroenterologischer Präparate zur Behandlung von Ulcera wie Ulcus duodenum verwendet.

Bei der Herstellung der erfindungsgemäßen wäßrigen synthetischen Organextrakte kann vorteilhafterweise zum einen die Verwendung von flüchtigen, meist brennbaren und oft toxischen Lösungsmitteln vermieden werden, zum anderen lassen sich die erfindungsgemäßen Organextrakte mit einem gleichbleibenden Qualitätsstandard herstellen, der bei herkömmlichen Extrakten nicht zuverlässig gegeben ist. Außerdem zeichnen sich die erfindungsgemäßen Organextrakte durch eine stark verbesserte Lagerfähigkeit aus. Während die erfindungsgemäßen Organextrakte vorzugsweise keine Konservierungsstoffe enthalten, lassen sich Naturextrakte im Regelfall nicht ohne Konservierungsmittel herstellen.

Die erfindungsgemäßen synthetischen Organextrakte sind wirksame Substitute oder Ergänzungen für eine Vielzahl von Naturstoffextrakten, insbesondere für Placenta-, Thymus-, Blut-, Blutserum-, Milz-, Leber-, Herzmuskel-, Elastin-, Kollagen-, Bindegewebs-, Amnionflüssigkeits-, Nabelschnur-, Quallen-, Rogen- und Euterextrakte sowie Kombinationen derselben.

Bevorzugt enthält der synthetische Organextrakt als wäßrige Lösung etwa 0,5 bis 50 g/l monomere Aminosäuren aus der Gruppe bestehend aus Glycin, Prolin, Hydroxyprolin, Serin, Lysin und Arginin. Im allgemeinen enthält die erfindungsgemäße Zusammensetzung Aminosäuregemische, welche geeigneterweise aus Proteinhydrolysaten bzw. Proteinpartialhydrolysaten, wie z.B. Sojabohnen-, Gluten-, Edestin- und Fibrinhydrolysaten, Hefeproteinhydrolysaten, partiell dehydratisierten Hefen und ihre Partialhydrolysate sowie Peptonen und Peptonhydrolysaten gewonnen werden. Die Konzentration der einzelnen Aminosäuren wird selbstverständlich durch ihre Löslichkeit begrenzt.

Erfindungsgemäß ist insbesondere ein hoher Anteil an Glycin, Prolin und Hydroxyprolin bevorzugt. Diese Aminosäuren sind im Kollagen in relativ großen Mengen enthalten und sie spielen hinsichtlich der Wirksamkeit der erfindungsgemäßen wäßrigen Organextrakte eine besondere Rolle. Kollagen selbst ist als vorteilhaftes Mittel zur Hautbehandlung allgemein bekannt.

Gemäß einer besonders bevorzugten Ausführungsform der Erfindung sind die monomeren Aminosäuren aus der Gruppe bestehend aus Glycin, Prolin, Serin, Lysin, Arginin und Hydroxyprolin vorzugsweise mit einem Anteil von 0,05 bis 5,0 Gew.% in der synthetisch wäßrigen Organextraktlösug enthalten.

Obwohl Histidin keine zwingende Aminosäurekomponente des erfindungsgemäßen synthetischen Organextraktes darstellt, ist seine Gegenwart besonders als Peptidaminosäure vorteilhaft, speziell dann, wenn Oligopeptide einen maßgeblichen Anteil der Peptidkomponente (b) bilden. Die Wirksamkeit von Histidin beruht möglicherweise u.a. auch auf dessen Komplexbindungsvermögen für Metallionen.

Gemäß einer bevorzugten Ausführungsform der Erfindung beträgt der Gesamtanteil der freien Aminosäuren in dem fertigen Extrakt 0,2 bis 6 Gew%.

Für Komponente (b) umfaßt der Begriff "Peptid" erfindungsgemäß Oligopeptide mit bis zu 10 verknüpften Aminosäuren sowie Polypeptide mit einer Kettenlänge von bis zu 100 verknüpften Aminosäuren. Erfindungsgemäß hat sich gezeigt, daß vor allem die Oligopeptide und Polypeptide mit Aminosäuren aus den Gruppen I, II und III die wirksamsten Aktivbestandteile der Peptidkomponente (b) sind.

Vorzugsweise enthält die Komponente (b) mindestens eine der Aminosäuren ausgewählt aus Serin, Arginin, Lysin, Prolin und Hydroxyprolin, vorzugsweise mindestens 2 der Aminosäuren Serin, Arginin, Lysin, Prolin, Hydroxyprolin, Histidin. Es ist wünschenswert, daß die Peptidkomponente (b) im wesentlichen Tri- bis Hexapeptide, Salze derselben und/oder Metallkomplexe derselben aufweist. In einer weiteren bevorzugten Ausführungsform der Erfindung kann sich die Komponente (b) mindestens teilweise aus Peptonen zusammensetzen, welche aus der Gruppe bestehend aus Sojabohnenpepton, Maispepton, Haferpepton, Hefepeptiden und/oder von partiell dehydratisierter Hefe ausgewählt sind.

Beispiele für Peptide, welche als Peptid-Grundlage für den erfindungsgemäßen Extrakt dienen können, sind N-Acetyl-L-Ala-L-Ala, N-Acetyl-L-Ala-L-Ala-L-Ala, N-Acetyl-D-Ala-D-Glu, N-Acetyl-L-Asp-L-Glu, N-Acetyl-L-Glu-Gly, N-Acetyl-Gly-Gly, N-Acetyl-Gly-L-Leu, N-Acetyl-L-His-Gly-L-His, N-Acetyl-L-Leu-Gly, N-Acetyl-L-Met-L-Ala-L-Ser, N-Acetyl-L-Met-L-Glu, N-Acetyl-L-Phe-L-Phe, N-Acetyl-L-Phe-L-Trp, N-Acetyl-L-Phe-L-Tyr, N-Acetyl-L-Pro-L-Leu-Gly, N-Acetyl-L-Ser-Gly, N-Acetyl-L-Tyr-L-Phe, N-Acetyl-L-Tyr-L-Tyr, N-Acetyl-muramyl-L-Ala-L-isoglutamin, L-Ala-L-Ala, β-Ala-β-Ala, L-Ala-L-Ala-L-Ala, L-Ala-L-Asn, L-Ala-L-Asp, L-Ala-L-Glu, L-Ala-L-Gln, L-Ala-Gly, L-Ala-Gly-L-Ala, β-Ala-Gly-L-Ala, L-Ala-L-His, β-Ala-L-His, β-Ala-L-Leu, L-Ala-L-Leu-L-Ala, L-Ala-L-Lys, L-Ala-L-Met, L-Ala-L-Nva, L-Ala-L-Phe, β-Ala-L-Phe, L-Ala-L-Phe-L-Ala, L-Ala-L-Phe-L-Pro, L-Ala-L-Pro, L-Ala-L-Pro-L-Ala, L-Ala-L-Ser, L-Ala-L-Ser-Gly, L-Ala-L-Thr, L-Ala-L-Tyr, L-Ala-L-Tyr-L-Ala, L-Ala-L-Val, L-Ala-L-Val-L-Leu, L-Abu-L-Tyr, L-Arg-L-Asp, L-Arg-L-Glu, L-Asn-L-Val, L-Asp-β-Ala, L-β-Asp-L-Ala, L-Asp-L-Glu, L-Asp-L-Gln, L-Asp-Gly, L-Asp-L-Leu, L-Asp-L-Lys, L-Asp-L-ε-Lys, L-Asp-L-Phe, L-Asp-L-Trp, L-Asp-L-Val, L-Cys-Gly, L-Cys-L-Ala-L-Ala, L-Cys-Gly-Gly, L-Cys-L-Leu-L-Leu, L-Cys-L-Phe-L-Phe, L-Cys-L-Pro-L-Pro, L-Cys-L-Tyr-L-Tyr, L-Cys-L-Val-L-Val, Gly-His-Lys, L-Gln-L-Val, L-Gln-L-Ala, L-γ-Glu-L-Ala, L-Glu-L-Ala-L-Ala, L-Glu-L-Asp, L-Glu-L-Glu, L-Glu-L-Glu-L-Glu, L-Glu-Gly, L-Glu-Gly-L-Phe, L-Glu-L-Lys, L-Glu-L-ε-Lys, L-γ-Glu-L-Lys, L-γ-Glu-L-Met, L-Glu-L-Ser, L-Glu-L-Thr, L-Glu-L-Thr-L-Tyr, L-Glu-L-Tyr, L-Glu-L-Tyr, L-Glu-L-Val, L-γ-Glu-L-Val, L-Glu-L-Val-L-Phe, Gly-L-Ala, Gly-DL-Ala, Gly-β-Ala, Gly-β-Ala-β-Ala, Gly-L-Ala-L-Asp, Gly-L-Ala-Gly, Gly-L-Ala-L-Hyp, Gly-L-Ala-L-Leu, Gly-L-Ala-L-Phe, Gly-L-Arg, Gly-L-Asn, Gly-L-Asp, Gly-L-Glu, Gly-Gly, Gly-Gly-L-Arg, Gly-Lys-His, Gly-Ala-Pro, Gly-His-Arg, Gly-Gly-L-Cys, Gly-Gly-Gly, Gly-Gly-Gly-Ala, Gly-Gly-Gly-Gly-Ala, Gly-Gly-L-His, Gly-Gly-L-Leu, Gly-Gly-L-Pro, Gly-Gly-L-Val, Gly-L-His, Gly-L-Hyp-L-Ala, Gly-L-Hyp-L-Ser, Gly-L-Ile, Gly-L-Met, Gly-DL-Nle, Gly-L-Phe, Gly-L-Phe-L-Ala, Gly-L-Phe-L-Leu, Gly-L-Phe-LPhe, Gly-L-Phe-L-Ser, Gly-L-Pro, Gly-L-Pro-L-Ala, Gly-L-ProL-Hyp, Gly-L-Pro-L-Pro, Gly-L-Ser, Gly-L-Ser-L-Ala, Gly-L-Thr, Gly-L-Trp, Gly-L-Tyr-L-Ala, Gly-L-Tyr-Gly, Gly-L-Val, L-His-LAla, L-His-L-Arg, L-His-L-Asp, L-His-Gly-L-Lys, L-His-L-Leu, L-His-L-Met, L-His-D-Phe, L-His-L-Pro, L-His-L-Ser, L-His-L-Trp, L-His-L-Trp-L-Lys, L-His-L-Tyr, L-His-L-Val, L-Hyp-Gly, L-IleL-Ala, L-Ile-L-Asn, L-Ile-L-Gln, L-Ile-Gly, L-Ile-Gly-Gly, L-Ile-L-His, L-Ile-L-Ile, L-Ile-L-Ile-L-Ile, L-Ile-L-Leu, L-Ile-L-Met, L-Ile-L-Phe, L-Ile-L-Ser, L-Ile-L-Trp, L-Ile-L-Tyr, L-Ile-L-Val, L-Leu-L-Ala, L-Leu-β-Ala, L-Leu-L-Ala-L-Pro, L-Leu-L-Asn, L-Leu-L-Asp, L-Leu-Gly, L-Leu-Gly-Gly, L-Leu-Gly-L-Leu, L-Leu-Gly-L-Pro, L-Leu-Gly-L-Tyr, L-Leu-L-His, L-Leu-L-Ile, L-Leu-L-Leu-L-Ale, L-Leu-L-Leu-Gly, L-Leu-L-Phe, L-Leu-L-Met, L-Leu-L-Phe, L-Leu-L-Ser, L-Leu-L-Tyr, D-Leu-L-Tyr, L-Leu-L-Val, L-Lys-L-Asp, L-Lys-L-Glu, L-Lys-L-Pro-L-Arg, L-Lys-L-Thr-L-Tyr, L-Lys-L-Trp, L-Lys-L-Tyr, L-Lys-L-Tyr-L-Glu, L-Lys-L-Tyr-L-Ser, L-Lys-L-Tyr-L-Thr, L-Met-L-Ala, L-Met-L-Ala-L-Ser, L-Met-L-Asn, L-Met-L-Asp, L-Met-L-Glu, L-Met-L-Gln, L-Met-Gly-Gly, L-Met-LHis, L-Met-L-His-Gly, L-Met-L-Ile, L-Met-L-Ile-Gly, L-Met-L-Leu, L-Met-L-Leu-Gly, L-Met-L-Met, L-Met-L-Met-L-Ala, L-Met-L-Met-L-Met, L-Met-L-Pro, L-Met-L-Pro-Gly, L-Met-L-Ser, L-Met-L-Ser-Gly, L-Met-L-Thr, L-Met-L-Tyr, L-Met-L-Val, L-Orn-L-Asp, L-Orn-L-Orn-L-Orn, L-Phe-L-Ala, L-Phe-β-Ala, L-Phe-L-Ala-L-Asn, L-Phe-L-Arg, L-Phe-L-Glu, L-Phe-L-Ile, L-Phe-L-Leu, L-Phe-L-Met, L-Phe-L-Phe, L-Phe-L-Phe-L-Phe, L-Phe-L-Pro, L-Phe-L-Ser, L-Phe-L-Ser-L-Val, L-Phe-L-Trp, L-Phe-L-Tyr, L-Phe-L-Val, Poly-L-Ala, Poly-Gly, Poly-L-Leu, Poly-L-Val, L-Pro-L-Ala, L-Pro-L-Arg, L-Pro-L-Asn, L-Pro-L-Asp, L-Pro-L-Glu, L-Pro-L-Gln, L-Pro-L-His-L-Ala, L-Pro-L-His-L-Asp, L-Pro-L-His-L-Glu, L-Pro-L-His-Gly, L-pro-L-His-L-Leu, L-Pro-L-His-L-Phe, L-Pro-L-His-L-Tyr, L-Pro-L-His-L-Val, L-Pro-L-Hyp, L-Pro-L-Ile, L-Pro-L-Leu, L-Pro-L-Met, L-Pro-L-Phe, L-Pro-L-Phe-L-Asp, L-Pro-L-Ser, L-Pro-L-Trp, L-Pro-L-Tyr, L-Pro-L-Tyr-L-Ala, L-ProL-Val, Sarcosyl-L-Ala, Sarcosyl-L-Asp, Sarcosyl-Gly, Sarcosyl-Gly-Gly, Sarcosyl-L-Ile, Sarcosyl-L-Leu, Sarcosyl-L-Met, Sarcosyl-L-Ser, Sarcosyl-L-Trp, Sarcosyl-L-Tyr, Sarcosyl-L-Val, L-Ser-L-Ala, L-Ser-β-Ala, L-Ser-L-Asp, L-Ser-L-Glu-Gly, L-Ser-Gly, L-Ser-Gly-Gly, L-Ser-L-His, L-Ser-L-Leu, L-Ser-L-Leu-L-Leu, L-Ser-L-Met, L-Ser-L-Phe, L-Ser-L-Pro, L-Ser-L-Ser, L-SerL-Ser-L-Ser, L-SerL-Tyr, L-Ser-L-Tyr-L-Lys, N-Succinyl-L-AlaL-Ala-L-Val, N-Succinyl-L-Pro, L-Thr-L-Ala, L-Thr-β-Ala, L-ThrL-Arg, L-Thr-L-Asn, L-Thr-L-Asp, L-Thr-L-Gln, L-Thr-Gly, L-Thr-Gly-Gly, L-Thr-L-Leu, L-Thr-L-Met, L-Thr-L-Pro-L-Lys, L-Thr-L-Ser, L-Thr-L-Tyr-L-Lys, L-Trp-L-Ala, L-Trp-L-Asp, L-Trp-L-Glu, L-Trp-Gly, L-Trp-Gly-L-Tyr, L-Trp-L-Leu, L-Trp-L-Pro, L-Trp-L-Ser, L-Trp-L-Trp, L-Trp-L-Trp-L-Trp, L-Trp-L-Tyr, L-Trp-L-Val, L-Tyr-L-Ala, L-Tyr-L-Ala-L-Phe, L-Tyr-L-Glu, L-Tyr-L-Gln, L-Tyr-L-Glu-L-Trp, L-Tyr-Gly, L-Tyr-Gly-L-Trp, L-Tyr-L-His, L-Tyr-L-Ile, L-Tyr-L-Leu, L-Tyr-L-Lys, L-Tyr-L-Lys-L-Thr, L-TyrL-Lys-L-Trp, L-Tyr-L-Phe, L-Tyr-L-Pro, L-Tyr-L-Thr-L-Leu, L-Tyr-L-Thr-L-Lys, L-Tyr-L-Trp, L-Tyr-L-Tyr, L-Tyr-L-Tyr-L-Leu, L-Tyr-L-Tyr-L-Phe, L-Tyr-L-Val, L-Tyr-L-Val, L-Val-L-Ala, L-Val-β-Ala, L- Val-L-Arg, L-Val-L-Asn, L-Val-L-Asp, L-Val-L-Glu, L-Val-Gly, L-Val-Gly-Gly, L-Val-L-Ile, L-Val-L-Leu, L- Val-L-Leu-L-Ser, L-Val-L-Lys, L-Val-L-Met, L-Val-L-Nle, L-Val-L-Phe, L-Val-L-Pro, L-Val-L-Pro-L-Leu, L-Val-L-Ser, L-Val-L-Trp-L-Ile, L-Val-L-Tyr, L-Val-L-Tyr-L-Pro, L-Val-L-Tyr-L-Val, L-Val-L-Val, L-Val-L-Val-L-Glu, L-Val-L-Val-L-Gln, L-Val-L-Val-Gly, L-Val-L-Val-L-Phe, L-Val-L-Val-L-Tyr,

sowie die Oligopeptide

N-Acetyl-Arg-Arg-Pro-Tyr-Ile-Leu, N-Acetyl-Asp-Arg-Val-Tyr-Ile-His-Pro-Phe-His-Leu, N-Acetyl-Asp-Arg-Val-Tyr-Ile-His-Pro-Phe-His-Leu-Leu-Val-Tyr-Ser, N-Acetyl-His-His-Gly-His, N-Acetyl-Pro-β-Ala-Pro-β-Ala-Pro-β-Ala-Pro-β-Ala, Ala-Ala-Ala-Ala-Glu-Glu-Glu, Ala-Ala-Ala-Ala-Glu-Glu-Glu-Glu-Glu, Ala-Ala-Ala-Ala-Tyr-Ala, Ala-Ala-Ala-Pro-Ala, Ala-Ala-Ala-Pro-Ala-Ala, Ala-Ala-Ala-Tyr-Ala, Ala-Ala-Ala-Tyr-Ala-Ala, Ala-Ala-Pro-Ala, Ala-Ala-Pro-Ala-Ala, Ala-Ala-Pro-Tyr-Ala, Ala-Ala-Tyr-Ala, Ala-Ala-Tyr-Ala-Ala, Ala-Arg-Pro-Ala-Lys, β-Ala-Arg-Ser-Ala-Pro-Thr-Pro-Met-Ser-Pro-Tyr, Ala-Gly-Gly-Asp-Ala-Ser-Gly-Gly, Ala-Gly-Gly-Gly, Ala-Gly-Gly-Gly-Gly, Ala-Leu-Ala-Gly, Ala-Leu-Ala-Leu, Ala-Pro-Arg-Val-Asp, L-Ala-L-Pro-L-Phe, Ala-Pro-Tyr-Ala, Ala-Ser-His-Leu-Gly-Leu-Ala-Arg, Arg-Arg-Gly-Asp-Met-Glu, Arg-Gly-Phe-Phe, Arg-Gly-Pro-Phe-Pro-Ile, Arg-Gly-Tyr-Ala-Leu-Gly, Arg-Gly-Val-Phe-Arg, Arg-Gly-Val-Phe-Arg-Arg, Arg-Tyr-Leu-Gly-Tyr-Leu, Arg-Tyr-Leu-Gly-Tyr-Leu-Glu, Asp-Ala-His-Lys, Asp-Ala-Ser-Gly-Glu, Asp-Ser-Asp-Pro-Arg, Asp-Tyr-Met-Gly, Gly-Arg-Gly-Asp, Gly-Gly-Phe-Leu, Gly-Gly-Phe-Met, Ala-Gly-Ser-Glu, Val-Gly-Asp-Glu, Val-Gly-Ser-Glu, Glu-Pro-Glu-Thr, Gly-Gln-Pro-Arg, Gly-Gly-Asp-Ala, Gly-Gly-Asp-Ala-Ser-Gly-Glu, Gly-Gly-Gln-Ala, Gly-Gly-Glu-Ala, Gly-Gly-Gly-Ser, Gly-Gly-His-Ala, Gly-Gly-His-Gly, Gly-Gly-Pro-Ala, Gly-Gly-L-Trp, Gly-Gly-Trp-Ala, Gly-Leu-Gly-Leu, Gly-Leu-Leu-Gly, Gly-Pro-Gly-Gly, Gly-Trp-Gly-Gly, Leu-Leu-Val-Phe, Leu-Leu-Val-Tyr, Leu-Trp-Met-Arg, Leu-Trp-Met-Arg-Phe, Lys-Ala-Phe-Gly, Lys-Glu-Thr-Tyr-Ser-Lys, Lys-Val-Glu-Gln-Glu-Gly-Tyr, Met-Cys-Glu-Lys, Met-Gly-Met-Met, Phe-Gln-Gly-Pro, Phe-Gly-Gly-Phe, Phe-Gly-Phe-Gly, Phe-Leu-Glu-Glu-Ile, Phe-Leu-Glu-Glu-Leu, Phe-Leu-Glu-Glu-Val, Phe-Met-Leu-Pro, Pro-Glu-Pro-Glu-Thr, Pro-Leu-Gly-Gly, Pro-Lys-Lys-Ala-Thr-Glu-Leu-Lys, Ser-Tyr-Ser-Met, Ser-Tyr-Ser-Met-Glu-His-Phe-Arg-Trp-Gly, Thr-Pro-Arg-Lys, Thr-Tyr-Ser-Lys, Trp-Gly-Gly-Gly-Tyr, Trp-Gly-Gly-Tyr, Trp-Pro-Pro-Pro-Tyr, Trp-Pro-Pro-Tyr, Tyr-Asp-Asp-Val-Glu-Ser-Asp-Gly-Ala-Val, Tyr-Glu-Glu-Trp, Tyr-Lys-Lys-Gly-Glu, Tyr-Pro-Phe-Pro, Tyr-Pro-Pro-Glu-Pro-Glu-Thr, Val-Ala-Ala-Phe, Val-His-Leu-Thr-Pro, Val-Leu-Ser-Glu-Gly, Val-Tyr-Ile-His-Pro.

Ferner sind Oligo-Alanine mit 2 bis 10 Alaninmolekülen, Oligo-Valine, Oligo-Glycine, Oligo-Phenylalanine und Oligo-Proline mit jeweils 2 bis 6 Aminosäuren als Bestandteile der Peptidgrundlage geeignet, wobei beispielsweise die C-terminalen Positionen dieser Homo-Oligopeptide mit heterologen Aminosäuren wie Glutaminsäure, Tyrosin, Lysin oder jeweils einer der oben genannten Aminosäuren besetzt sein können. Ferner können diese Oligopeptide in N-terminaler Position acetyliert sein.

Bei der Herstellung von synthetischen Placentaextrakten haben sich insbesondere die Peptidfragmente Gly-His-Lys, Gly-His-Pro und Glutathion sowie Arg-Gly-Asp-Ser, Gly-Arg-Gly-Asp, Gly-Arg-Gly-Asp-Ser, Gly-Arg-Gly-Asp-Ser-Pro, Gly-Arg-Gly-Asp-Ser-Pro-Lys und Gly-Arg-Gly-Asp-Thr-Pro als vorteilhaft erwiesen.

Bei der Herstellung eines kombinierten synthetischen Serum-/Placentaextraktes werden bevorzugt die Peptidfragmente Arg-Gly-Val-Phe-Arg-Arg, Arg-Gly-Val-Phe-Pro, Arg-Gly-Val-Phe-Ser, Arg-Gly-Phe-Arg, Arg-Gly-Val-Phe-Pro, Arg-Gly-Val-Phe-Arg-Val-Arg und Arg-Gly-Val-Phe-Arg-Pro verwendet.

Bei der Herstellung von synthetischen Thymusextrakten werden bevorzugt die Peptidfragmente Gly-Pro-His, Gly-Lys-His, Gly-His-Lys, Gly-Ala-His, Glutathion, Gly-Pro-Hyp, Gly-Val-His, Gly-His-Pro, sowie Gly-His-His-Gly-His-Lys, Gly-Pro-Hyp-Gly-His-Val und Gly-Pro-His-Gly-Pro-His verwendet.

Kollagene und Elastine werden in der Kosmetik seit vielen Jahren verwendet. Es hat sich gezeigt, daß sich einzelne, ausgewählte Oligopeptide, die in der Sequenz von Proteinen wie z.B. Kollagenen, Elastinen, Keratinen, und Fibronektinen vorkommen, und die synthetisch erhältlich sind, für die Wirkung der erfindungsgemäßen synthetischen wäßrigen Organextrakte besonders eignen.

Das in der Kollagensequenz enthaltene Tripeptid Glycyl-L-Histidyl-L-Lysin hat sich als besonders vorteilhaft erwiesen, da es in Fibroblasten-Kulturen die Kollagensynthese stimuliert und eine wichtige Rolle bei der Wundheilung spielt.

Die Peptidkomponente (b) kann zwar ausschließlich von synthetischen Peptiden gebildet werden. Es wird jedoch bevorzugt, daß die Peptide zumindest teilweise durch schonende Hydrolyse oder Partialhydrolyse von höheren Peptiden oder Proteinen mittels Mineralsäuren, Natronlauge und/oder Enzymen, und gegebenenfalls nach Selektion durch Ultrafiltration, gewonnen werden. In diesen Fällen ist jedoch stets darauf zu achten, daß keine immunogenen bzw. pathogenen Proteine und Proteinabbauprodukte sowie insbesondere keine unkonventionellen Viren und Prionen bzw. deren Proteinbestandteile in den synthetischen Organextraktansatz gelangen.

Nach einer bevorzugten Ausführungsform der Erfindung setzt sich die Peptidkomponente (b) mindestens teilweise aus Peptonen zusammen, welche aus der Gruppe bestehend aus Gelatinepepton, Sojabohnenpepton, Maispepton, Haferpepton, Fischpepton, Fischkollagen, Quallenkollagen, Säuretierkollagen, Kollagenpepton, Caseinpepton, Hefepeptiden und/oder von einer partiell dehydratisierten Hefe mit einem Wassergehalt von 60 bis 80% ausgewählt sind. Weiterhin ist es bevorzugt, daß der synthetische Organextrakt zusätzlich Albumin enthält.

In einem erfindungsgemäß bevorzugten synthetischen Organextrakt setzt sich die Komponente (b) mindestens teilweise aus Peptonen zusammen, welche aus der Gruppe bestehend aus Sojabohnenpepton, Maispepton, Haferpepton, Hefepeptiden und/oder von partiell dehydratisierter Hefe ausgewählt sind.

Die Dominanz der vorstehend diskutierten Aminosäuren bedeutet im Hinblick auf die Zusammensetzung der erfindungsgemäßen synthetischen Extrakte, daß die Komponente (b) bestimmte Aminosäuren wie z.B. Serin, Arginin, Lysin, Prolin und/oder Hydroxyprolin in Mengen enthält, die weit über dem Gehalt dieser Aminosäuren in dem entsprechenden Naturextrakt liegen, d.h. in einer 2- bis 15-fachen Menge. Insbesondere bei der Peptidkomponente (b) und speziell bei den in ihr enthaltenen Oligopeptiden hat sich gezeigt, daß Peptide, die mindestens zwei der Aminosäuren Serin, Arginin, Lysin, Prolin, Hydroxyprolin und Histidin aufweisen, besonders aktiv sind.

Andere Aminosäuren können in monomerer und/oder petidgebundener Form anwesend und beispielsweise aus der folgenden Zusammenstellung ausgewählt sein.

Geeignete Aminosäuren und Aminosäurederivate sind Asparagin, Asparaginsäure, Cystein, Cystin, Glutaminsäure, Glutamin, alpha-Alanin, beta-Alanin, Arginin, Glycin, Histidin, delta-Hydroxylysine, Hydroxyproline, Leucin, Isoleucin, Lysin, Methionin, Norleucin, Phenylalanin, Prolin, Serin, Threonin, Tryptophan, Tyrosin, Valin, alpha-Aminoadipinsäure, alpha-Aminobuttersäure (normal), gamma-Amino-n-buttersäure, beta-Amino-isobuttersäure, delta-Aminolävulinsäure, Carbamylasparaginsäure, Citrullin, Kreatin, Kreatinin, Cystathionin, Cysteinsäure, Ergothionein (Betain des Thiolhistidins), Glycocyamin (Guinidinessigsäure), Homoserin, Ornithin, Taurin, Djenkolsäure (Cysteinthioformacetal), Guanidinsalze, Ornithursäure, Phenacetursäure, Hippursäure, Harnstoff, N-Acetyl-L-alanin, N-Acetyl-L-arginin, N-Acetylglycin, N-Acetyl-L-hydroxyprolin, N-Acetyl-L-isoasparagin, N-Acetyl-L-isoleucin, N-Acetyl-L-leucin, N-Acetyl-L-lysin, N-Acetyl-L-methionin, N-Acetylmuraminsäure, N-Acetyl-L-ornithin, N-Acetyl-L-phenylalanin, N-Acetyl-L-prolin, 0-Acetyl-L-serin, N-Acetyl-L-threonin, N-Acetyl-L-tryptophan, N-Acetyl-L-valin, L-allo-Isoleucin , L-allo-Threonin , N-Benzoyl-D-alanin, N-Benzoyl-L-arginin, N-Benzoyl-L-histidin, N-Benzoyl-L-lysin, N-Benzoyl-L-methionin, N-Benzoyl-L-ornithin, N-Benzoyl-L-phenylalanin, N-Benzoyl-L-tryptophan, N-Benzoyl-L-valin, S-Benzoyl-L-cystein, O-Benzoyl-L-serin, O-Benzoyl-L-tyrosin, L-Carnosin ($\beta$-Alanyl-L-histidin), N,O-Diacetyl-L-threonin, O-Phospho-L-serin, O-Phospho-D-serin.

Gemäß einer besonders bevorzugten Ausführungsform der Erfindung enthält die Komponente (b) im wesentlichen Tri- bis Hexapeptide, Salze und/oder Metallkomplexe derselben. Vorzugsweise beträgt der Gesamtanteil der Peptidkomponente (b) 0,1 bis 6 Gew.%, bezogen auf den Gesamtextrakt.

Vorteilhaft ist, wenn der erfindungsgemäße synthetische Organextrakt neben den Aminosäuren und Peptiden noch eine zusätzliche Stickstoffquelle enthält. Hierzu gehören insbesondere Harnstoff, Cysteamin und N-Methylglucamin. In manchen Fällen ist auch die Anwesenheit von Kreatinin günstig, vorzugsweise in einer Menge von 0,001 bis 0,005 Gew.%.

Die Komponente (c) umfaßt Purin- und Pyrimidinbasen (Nucleobasen), Nucleostide, Nucleotide sowie deren Derivate und Nucleinsäuren.

Beispiele für geeignete Verbindungen aus dieser Gruppe sind 2-Aminopurin, Hypoxanthin, 1-Methylhypoxanthin, Adenin, 2-Methyladenin, 6-Methylaminopurin, Guanin, 1-Methylguanin, 7-Methylguanin, 2-Methylamino-6-oxodihydropurin, 8-Hydroxyguanin, Isoguanin, 2,6-Diaminopurin, Xanthin, 1-Methylxanthin, 3-Methylxanthin, 7-Methylxanthin, 3,9-Dimethylxanthin, Harnsäure, 1-Methylharnsäure, 9-Methylharnsäure, 1,9-Dimethylharnsäure, 3,7-Dimethylharnsäure, 1,3,7-Trimethylharnsäure, Adenosin, 3'-Amino-3'-desoxy-adenosin, 9-$\beta$-D-Ribofuranosyl-6-methylaminopurin, 2'-Desoxyinosin, 2'-Adenylsäure, 3'-Adenylsäure, 5'-Adenylsäure, Adenosin-5'-diphosphorsäure, Adenosin-5'-triphosphorsäure, Desoxyadenylsäure, 2'-Desoxyadenosin-5'-triphosphat, N-Succinyladenylsäure, 3'-Guanylsäure, Guanosin-5'-diphosphorsäure, Guanosin-5'-triphosphorsäure, Inosin-3'-phosphorsäure, Inosin-5'-phosphorsäure, Inosin-5'-diphosphorsäure, Xanthylsäure, Xanthosin-5'-monophosphorsäure, Guanosindiphosphat-mannose, Guanosindiphosphat-fructose, Guanosindiphosphat-fucose, Diphosphopyridinnucleotid, Triphosphopyridinnuelotid u.a., Cytosin, 5-Methylcytosin, 5-Hydroxymethylcytosin, Cytimidin, Thymin, Uracil, Willardiin, 5-Aminouracil, 4,5-Dihydrouracil, 4-Aminouracil, Uridin, 4,5-Dihydrouridin, 2'-Desoxyuridin, 5-Methyluridin, Pseudouridin, Orotidin, Cytidin, 2'-Desoxycytidin, 5-Methylcytidin, Thymidin, a-Thymidin, Cytidin-2'-monophosphat, Cytidin-3'-monophosphat, Cytidin-5'-monophosphat, Cytidin-5'-diphosphat, Uridin-2'-monophosphat, Uridin-3'-monophosphat, Uridin-5'-monophosphat, Uridin-5'-diphosphat, 5-Methylcytidin-3'-monophosphat, Orotidin-5'-monophosphat, Thymidin-5'monophosphat, Thymidin-5'-triphosphat, 2'-Desoxycytidin-5'monophosphat, 2'-Desoxycytidin-5-diphosphat, Uridindiphosphat-glucose, Uridindiphosphatgalaktose, Uridindiphosphat-arabinose, Uridindiphosphat-xylose, Uridindiphosphat-glucuronsäure, Uridindiphosphat-N-acetylgalaktosamin, Cytidindiphosphat-a-glycerin, Cytidindiphosphat-ribitol, cycl. AMP, cycl. GMP u.a.

Als Komponente (c) werden bevorzugt Inosin, Adenin, Adenosin, Guanosin, cycl. AMP und/oder AMP eingesetzt, wobei deren Gesamtmenge in einer wäßrigen Endlösung des synthetischen Gesamtextraktes 0,001 bis 0,25 Gew% betragen kann.

Als weitere wesentliche Komponente (d) der erfindungsgemäßen synthetischen Organextrakte ist mindestens ein Kohlenhydrat und/oder Kohlenhydratderivat enthalten, wobei die Komponente (d) mindestens einen Zuckeralkohol aus der Gruppe bestehend aus Sorbit, Mannit, Inosit und Dulcit in einer Menge von mindestens 0,2 Gew%, bezogen auf das Endprodukt, umfaßt.

Beispiele für andere geeignete Verbindungen der Gruppe (d) sind Dihydroxyaceton, Dihydroxyacetonphosphate, D-Glycerinaldehyd, D-Glycerinaldehyd-3-phosphat, D-Erythrose, β-D-Arabinose, D,L-Arabinose, 2-Desoxy-D-ribose (Desoxyarabinose), L-Fructose (6-Desoxy-L-galactose), L-Rhamnose (6-Desoxy-L-mannose), D-Ribose, D-Ribulose, D-Xylulose, L-Xylulose, D-Fructose, D-Galactose, D-Galactosamin (2-Amino-2-desoxy-D-galactose), N-Acetyl-D-galactosamin, D-Glucose, D-Glucosamin, N-Acetyl-D-glucosamin, N-Methyl- L-glucosamin, D-Mannose, D-Seduheptulose, L-Glycerin-1-phosphat, L-Glycerinsäure-2-phosphat, D-Glycerinsäure-3-phosphat, D-Glycerinsäure-1,3-diphosphat, D-Glycerinsäure-2,3-diphosphat, Phosphoenolbrenztraubensäure, D-Erythrose-4-phosphat, L-Erythrulose-1-phosphat, alpha-D-Ribose-1-phosphat, D-Ribose-5-phosphat, Desoxyribose-1-phosphat, D-Ribulose-5-phosphat, D-Xylulose-5-phosphat, D-Fructose-1-phosphat, D-Fructose-6-phosphat, D-Fructose-1,6-diphosphat, alpha-D-Galactose-1-phosphat, D-Galactosamin-1-phosphat, N-Methylgalactosamin, N-Methylglucosamin, alpha-D-Glucose-1-phosphat, D-Glucose-6-phosphat, β-D-Glucose-1,6-diphosphat, D-Glucosamin-6-phosphat, D-Gluconsäure-6-phosphat, D-Mannose-6-phosphat, D-Seduheptulose-7-phosphat, D-Seduheptulose-1,7-diphosphat, Lactosephosphat, Cellobiose, Maltose, Saccharose, Lactose, Fucosidolactose, Gentiobiose, Trehalose, Disacchararid aus Glucuronsäure und N-Acetylglucosamin.

Von diesen Kohlenhydraten und Derivaten werden Glucose, Invertzucker, D-Mannose, D-Ribulose, L-Erythrulose-1-phosphat, D-Fructose-6-phosphat, D,L-Arabinose und die N-Methylhexosamine bevorzugt verwendet.

Die Kohlenhydrate können als Einzelverbindungen oder als Gemische von Kohlenhydraten, wie z.B. als Hydrolysate von Melassen, Stärken, Glykogen, Inulin, Agar-Agar und Alginaten eingesetzt werden.

Zu den Kohlenhydratderivaten gehören neben den Zuckeralkoholen auch die Oxidationsprodukte von Kohlenhydraten, wie D-Glycerinsäure, Isoascorbinsäure, L-Ascorbinsäure, Dehydroascorbinsäure, 2,3-Diketogluconsäure, D-Gluconsäure, alpha-D-Galacturonsäure, β-D-Glucuronsäure, D-Iduronsäure, Zuckersäure, die Dicarbonsäure der Mannose und Galactose, wobei L-Ascorbinsäure, D-Glycerinsäure und D-Iduronsäure besonders bevorzugt sind.

Der Bedeutung der genannten Zuckeralkohole entsprechend kann in bevorzugten synthetischen Organextraktzusammensetzungen die Kohlenhydratkomponente (d) Sorbit und/oder Mannit in einer Menge enthalten, die einem Vielfachen des in dem vergleichbaren natürlichen Organextrakt vorkommenden Gesamtkohlenhydratgehaltes entspricht. Dieses Vielfache kann bis zu einem Faktor von 100 reichen.

Ein Anteil an Komponente (d), insbesondere an Hexiten von 0,2 bis zu 8 Gew.% hat sich als vorteilhaft erwiesen.

Aliphatische Carbonsäuren mit 3 bis 6 Kohlenstoffatomen bilden die Komponente (e) des erfindungsgemäßen synthetischen Organextraktes. Verbindungen aus dieser Gruppe sind in lebenden Zellen nachweisbar. Hierzu gehören Milchsäure, Äpfelsäure, Bernsteinsäure, alpha-Ketoglutarsäure, Citronensäure, iso-Citronensäure, cis-Aconitsäure, Fumarsäure, Oxalessigsäure, die Weinsäuren, Brenztraubensäure, Mevalonsäure, Acetessigsäure, β-Hydroxybuttersäure und Orotsäure. Bevorzugt werden Citronensäure, Bernsteinsäure, Milchsäure, alpha-Ketoglutarsäure, cis-Aconitsäure und Brenztraubensäure. In einer bevorzugten Ausführungsform der Erfindung wird dem synthetischen Organextrakt Bernsteinsäure und/oder Äpfelsäure mit einem Anteil von 0,02 bis 1,5 Gew.% zugesetzt. Neben Bernsteinsäure wird auch durch Citronensäure die Radikalfängerwirkung von Tocopherolen positiv beeinflußt.

Die Alkoholkomponente (f) des erfindungsgemäßen synthetischen Organextraktes umfaßt nichttoxische aliphatische und/oder aromatische Alkohole mit 2 bis 7 Kohlenstoffatomen, wie Ethanol, Butanol, Glycerin und Benzylalkohol. Benzylalkohol kann allein oder in Kombination mit weiteren Alkoholen aus Gruppe (f) verwendet werden. Die Alkoholmenge beträgt mindestens 0,3 Gew.%. Der verwendete Benzylalkohol kann in geeigneter Weise als Lösungsvermittler zur Herstellung der erfindungsgemäßen Organextrakte dienen.

Erfindungsgemäß bevorzugte Alkohole sind Benzylalkohol, Glycerin und Ethanol.

Neben den vorstehend erläuterten wesentlichen Komponenten des erfindungsgemäßen Präparats können gegebenenfalls noch Vitamine wie beispielsweise D-Panthenol, Mineralsalze und/oder Spurenelemente sowie Puffersubstanzen und Konservierungsstoffe enthalten sein, wobei deren Mengen im üblichen Rahmen liegen. Die gegenüber natürlichen Extrakten größere Pufferkapazität der erfindungsgemäßen synthetischen Organextrakte wird neben den obengenannten Aminosäuren außerdem vorteilhafterweise durch die Verwendung bestimmter Säuren wie Citronensäure und Milchsäure bestimmt, wobei der pH-Wert der Lösung auf etwa 4,0 bis 7,0 eingestellt bzw. dieser Wert aufrechterhalten wird. Für Panthenolhaltige synthetische Organextrakte liegt der pH-Wert im unteren Teil des angegebenen Bereichs.

Der erfindungsgemäße synthetische Organextrakt kann üblicherweise verwendete Konservierungsmittel enthalten; vorzugsweise ist er jedoch frei von derartigen Mitteln.

Die Verwendung von Ascorbinsäure bzw. Ascorbat ist für den erfindungsgemäßen synthetisch wäßrigen Organextrakt von besonderer Bedeutung. Schon kleine Mengen zeigen bei pH 6,5 - 6,8 Radikalfängereigenschaften und sind hinsichtlich der Stabilität solcher Organextrakte von Vorteil. In diesem Zusammenhang haben sich auch Tocopherolsuccinat, -acetat und -nicotinat als besonders vorteilhaft erwiesen. Die Wirkung von Tocopherolen wird durch kleine Mengen Citronen- oder Bernsteinsäure positiv unterstützt. Auch sind geringe Mengen 2-Thioxanthin als Radikalfänger für die erfindungsgemäßen Organextrakte geeignet.

Erfindungsgemäß ist es auch möglich, daß mindestens ein Teil der Komponenten von einem entsprechenden dialysierten entproteinierten Organextrakt-Hydrolysat gebildet wird, insbesondere von solchen Organextrakt-Hydrolysaten, deren synthetisches Gegenstück angestrebt wird. In diesem Fall dient die erfindungsgemäße Präparation als Supplement des jeweiligen Naturstoffextraktes.

Gemäß einer bevorzugten Ausführungsform betrifft die Erfindung einen synthetischen wäßrigen Organextrakt worin die Aminosäurekomponente (a) und die Peptidkomponente (b) etwa

- 0,2 bis 0,7 Gew.% Aminosäuren der Gruppe (I),
- 0,05 bis 0,15 Gew.% Aminosäuren der Gruppe (II) und
- 0,10 bis 0,20 Gew.% Aminosäuren der Gruppe (III)

  umfassen und

- die Komponente (c) mit einem Anteil von 0,02 bis 0,06 Gew.%,
- die Komponente (d) mit einem Gesamtanteil an Sorbit, Mannit und Inosit von 0,2 bis 0,5 Gew.%,
- die Komponente (e) mit einem Anteil von 0,2 bis 0,7 Gew.% und
- die Komponente (f) mit einem Anteil von 0,3 bis 0,7 Gew.%

  jeweils bezogen auf den Gesamtextrakt in der synthetischen Extraktlösung enthalten ist.

Gemäß einer besonders bevorzugten Ausführungsform der Erfindung wird der synthetische wäßrige Organextrakt modifiziert, indem in dem zuvor genannten Extrakt die Menge der Aminosäuren der Gruppe (I) auf 2 bis 6 Gew.% und die Menge der Komponente (d) auf 3 bis 8 Gew.% erhöht werden, wobei die Mengen der übrigen Bestandteile unverändert bleiben. Ferner kann als Vitaminkomponente (g) D-Panthenol in einer Menge von bis zu 50 Gew.%, insbesondere von 15 bis 50 Gew.% und besonders bevorzugt von 24 bis 36 Gew.%, zugesetzt werden.

In einem derartigen, bevorzugten Extrakt kann der Anteil der Aminosäuren aus der Gruppe (I) durch Zugabe von 1,5 bis 4,5 Gew.%, vorzugsweise 2,4 bis 3,6 Gew.% Glycin und der Anteil der Komponente (d) (Zuckeralkohol) beispielsweise durch Zugabe von 3,0 bis 7,0, vorzugsweise 4 bis 6 Gew.% D-Mannit erhöht werden.

Dem Extrakt wird vorzugsweise ferner D-Lactose-Monohydrat als Stabilisator für D-Panthenol in einem Anteil von 0,8 bis 1,2 Gew.% zugesetzt.

Die Erhöhung des Anteils an Glycin auf 1,5 bis 4,5 Gew.%, vorzugsweise 2,4 bis 3,6 Gew.%, hat sich zur Verbesserung der Epithelisierung der Haut bzw. der Förderung der Wundheilung besonders bewährt. Durch den erhöhten Anteil an D-Mannit lassen sich die Eigenschaften der synthetisch wäßrigen Organextrakte weiter verbessern, und es hat sich erwiesen, daß die erfindungsgemäß definierte Wirkstoff-Kombination in den oben angegebenen Mengenverhältnissen in Verbindung mit den übrigen Komponenten die Wirksamkeit und das Wirkungsspektrum der synthetischen Organextrakte in hohem Maße positiv beeinflußt.

Die getrennte oder kombinierte Verwendung von D-Panthenol, Glycin und D-Mannit, vorzugsweise mit den oben genannten Anteilen, führt zu wäßrigen synthetischen Organextrakten, die in ihrer biologischen Funktion und Wirksamkeit verbesserte Eigenschaften aufweisen. Insbesondere zeichnen sich die synthetischen Organextrakte durch eine Erhöhung der Zellproliferation und eine positive Beeinflussung des Keratinisierungsprozesses aus. Eine noch weiter beschleunigte Wundheilung, verbunden mit einer Förderung der Granulation sowie eine Stimulierung der Epithelisierung sind neben einer Verbesserung des Feuchthaltevermögens der Haut (Moisturising effect), einer Normalisierung des Fettgehaltes sowie einer besseren Schutzwirkung vor Radikalen, besonders in hydrophilen inter- und intrazellulären Bereichen, als besondere Vorteile der erfindungsgemäßen synthetischen wäßrigen Organextrakte zu beobachten. Die Formulierungen führen zur Durchblutungsförderung und zur allgemeinen Linderung von Hautreizungen. Es kommt somit nicht nur zur Verminderung des Erythems und zur Verbesserung der Hautregeneration nach Sonnenbrand, sondern es wird eine noch weitere, allgemeine Verbesserung des Hautzustandes, wie zum Beispiel Hautglättung und Geschmeidigkeit der Haut, erreicht.

Bei Verwendung von D-Panthenol, das Vorstufe der im Haar vorkommenden D-Pantothensäure ist, eignen sich die wäßrigen synthetischen Organextrakte außerdem vorteilhaft für den Einsatz in der Haarkosmetik. Die Kombination der in den erfindungsgemäßen Organextrakten enthaltenen Inhaltsstoffe sorgt bei gleichzeitiger Anwesenheit von Panthenol für die Ausbildung einer Schutzschicht, die das Haar in äußerst vorteilhafter Weise vor dem Austrocknen schützt.

Die erfindungsgemäßen Organextrakte sind im wesentlichen proteinfrei, und vorzugsweise wird gänzlich auf Proteine verzichtet.

Der Trockenstoffgehalt der synthetischen Organextraktlösung kann, abhängig vom Verwendungszweck, 0,2 bis 60 Gew.% betragen. Der Trockenstoffgehalt des oben erläuterten, besonders bevorzugten Extraktes kann 30 bis 50 Gew.% betragen.

Die erfindungsgemäßen synthetischen wäßrigen Organextrakte können als Ersatz oder als Ergänzung für Placenta-, Thymus-, Blut-, Milz-, Leber-, Kollagen-, Bindegewebs-, Amnionflüssigkeits-, Quallen-, Rogen- und Euterextrakte bzw. für deren Kombinationen verwendet werden.

Sie sind besonders zur Herstellung von kosmetischen Formulierungen geeignet, wobei zwei oder mehrere Typen miteinander vereinigt werden können, um spezielle Effekte zu erzielen.

Die erfindungsgemäßen synthetischen wäßrigen Organextraktes sind ferner zur Herstellung medizinischer Präparate zur topischen, subkutanen oder intramuskulären Applikation für die Zwecke der äußeren und inneren Wundheilung geeignet sowie zur Herstellung medizinischer Präparate zur Stärkung des Immunsystems.

Schließlich stellen sie wertvolle Mittel zur Herstellung medizinischer Präparate zur Aktivierung des Zellstoffwechsels und zur Behandlung gastroenterologischer Erkrankungen, insbesondere von Ulcera dar.

Für die Herstellung der hier beschriebenen synthetischen Organextraktpräparate hat sich ein Verfahren besonders bewährt, bei dem man zunächst ein Gemisch aus zwei Teillösungen herstellt, wobei die Teillösung I eine wäßrige, entionisierte Lösung ist, welche die Hexite, Kohlenhydrate, leicht wasserlöslichen Aminosäuren bzw. deren Salze und gegebenenfalls Harnstoff und Konservierungsstoffe enthält, und die Teillösung II eine alkalischwäßrige Lösung der in Alkali leicht löslichen Aminosäuren ist. Das alkalisch reagierende Gemisch wird dann mit den Carbonsäuren und Alkoholen versetzt, bevor die Mineralsalze, Vitamine und Spurenelemente hinzugegeben werden. Danach erfolgt die Zumischung der wasserlöslichen bzw. wasserlöslich gemachten Nucleinsäureverbindungen. Dieser Ansatz wird schließlich mit einem oder mehreren desodorierten Ansätzen von die Peptidkomponente (b) enthaltenden, gegebenenfalls zuvor entsalzten, wäßrigen Lösungen vereinigt und mit Wasser auf das Endvolumen eingestellt. Der pH-Wert der Endlösung wird vorzugsweise auf 4,0 bis 7,0 eingestellt. Abschließend wird die Lösung sterilfiltriert. Als besonders vorteilhaft bei der Herstellung der erfindungsgemäßen Extrakte hat sich die Verwendung von vollständig entsalztem bzw. bidestilliertem Wasser erwiesen.

Es ist zweckmäßig, die Mischungs- und Lösungsschritte unter stetigem Rühren und unter Stickstoff-Schutzatmosphäre durchzuführen. Die Peptidlösungen werden vorzugsweise mit Aktivkohle desodoriert. Das Arbeiten unter pyrogenfreien Bedingungen schafft die besten Voraussetzungen für den Erhalt von stabilen synthetischen Organextraktlösungen und ist deshalb besonders bevorzugt.

Zur Herstellung eines erfindungsgemäß bevorzugten Extraktes kommen Komponenten aus den Gruppen (a) - (k) in den nachfolgend angegebenen Zusammensetzungen und Mengenanteilen in Betracht.

(a) <u>Aminosäuren und Derivate</u>

| | |
|---|---|
| L-Glutaminsäure | 0,1 - 2,5 g/l |
| L-Histidin, L-Ornithin.HCl, L-Asparagin, L-Asparaginsäure, L-Valin, L-Tyrosin, L-Phenylalanin, Kreatinin | je 0,01 - 0,5 g/l |
| Hydroxyprolin | 0,3 - 1,2 g/l |
| DL-Threonin | 0,03 - 0,5 g/l |
| L-Methionin, L-Isoleucin, L-Tryptophan, L-Leucin, $\beta$-Alanin, Cystein | je 0,01 - 0,07 g/l |
| L-Alanin, Glycin, L-Prolin, L-Serin, L-Arginin • HCl, L-Lysin • HCl | je 0,15 - 40,0 g/l |
| Hippursäure | 0,0005 - 0,8 g/l |
| Harnstoff | 0,15 - 10,0 g/l |

(b) <u>Peptide und Derivate</u>

| | |
|---|---|
| Oligopeptide mit 3 bis 6 Aminosäuren, ggfs. als Metallkomplexe stabilisiert | 0,01 - 5,0 mg/l |

Weitere Elemente der Komponentgruppen (a) und (b) können durch Peptonzusätze bzw. durch nachfolgend näher erläuterte Zusätze in die Endlösung gelangen.

(c) <u>Nucleinsäure-Komponenten und Derivate</u>

| | |
|---|---|
| Adenin, Adenosin, Cytidin, Cytosin, Guanin, Guanosin, Hypoxanthin, Inosin, Thymin, Uracil, Uridin, Xanthin, Harnsäure, Orotsäure, cycl. AMP, und/ oder Adenosinmonophosphat | je 0,0005 - 0,2 g/l |

(d) <u>Kohlenhydrate und Derivate</u>

| | |
|---|---|
| Glucose | 0,01 - 1,0 g/l |
| Sorbit, Mannit, Inosit und/oder Dulcit | je 0,2 - 60,0 g/l |

(e) <u>Aliphatische Carbonsäuren</u>

| | |
|---|---|
| Bernsteinsäure, Äpfelsäure und/oder Citronensäure | je 0,25 - 10,0 g/l |

(f) <u>Alkohole mit 2 bis 7 Kohlenstoffatomen</u>

| | |
|---|---|
| Ethanol (96%-ig, medizinisch rein) | 1,0 - 25,0 g/l |
| Glycerin | 0,25 - 5,0 g/l |
| Benzylalkohol | 0,05 - 3,0 g/l |

(g) <u>Vitamine</u>

| | |
|---|---|
| Thiamindichlorid, Nicotinsäureamid, Nicotinsäure, p-Aminobenzoesäure, Calciumpanthotenat, myo-Inosit, Pyridoxol · HCl, Biotin und/oder Tocopherolsuccinat | je 0,1 - 50 mg/l |

und/oder

| | |
|---|---|
| D-Panthenol | 200 - 400 g/l |

(h) <u>Mineralsalze und Spurenelemente</u>

gegebenenfalls

| | |
|---|---|
| Magnesiumsulfat, Magnesiumacetat, Magnesiumaspartat | je 0,002 - 0,1g/l |
| Zinkacetat, Kobaltgluconat, Mangangluconat, Zinkchlorid, Kupferacetat | je 0,001 -0,1mg/l |

(i) Sonstige Bestandteile

| N-Methylglucamin | 0,1 - 3,0 g/l |
|---|---|
| Natriumlactat | 0,1 - 5,0 g/l |

und gegebenenfalls

| Lactose-Monohydrat | 5,0 - 15,0 g/l |
|---|---|

(k) Konservierungsstoffe

| p-Hydroxybenzoesäuremethylester-Natriumsalz | 1,0 - 2,5 g/l |
|---|---|

und/oder

| Kaliumsorbat | 0,1 - 5,0 g/l |
|---|---|

Die Komponente (k) umfaßt erfindungsgemäß sämtliche der in Anlage 6 der Kosmetik-Verordnung vom 19. Juni 1985 (BGBl. I S. 1082), zuletzt geändert am 21.3.1990 (BGBl. I S. 589), angegebenen Konservierungsstoffe.

Die Teillösung I kann hergestellt werden, indem die folgenden Bestandteile unter sterilen Bedingungen und unter Rühren in bidestilliertem Wasser entsprechend 3/10 bis 4/10 des Endvolumens bei einem pH-Wert zwischen 6,0 und 7,0 gelöst werden. Hierzu werden zunächst die Aminosäuren L-Glutaminsäure, L-Asparaginsäure, L-Valin, L-Tyrosin, L-Phenylalanin, L-Isoleucin und L-Leucin in 2 N NaOH vorgelöst. Anschließend erfolgt die Zugabe der Carbonsäuren Citronensäure, Bernsteinsäure und Äpfelsäure, wobei der pH-Wert gegebenenfalls nachreguliert werden muß. Zu diesem Ansatz werden dann die weiteren Aminosäuren bzw. deren Derivate, nämlich L-Alanin, L-Histidin, Glycin, L-Ornithin, L-Asparagin, L-Threonin, L-Hydroxyprolin, Kreatinin, L-Methionin, L- Tryptophan, L-Prolin, L-Serin, L-Arginin • HCl, L-Lysin.HCl sowie Hippursäure und Harnstoff gegeben. Die Nucleinsäurekomponenten werden teilweise vorgelöst, so z.B. Xanthin in 3N NaOH und Guanin in 3N HCl, und dann vor deren Zugabe weitgehend neutralisiert. Die jeweiligen Mengen der für eine gewünschte Teillösung I benötigten Komponenten variieren in Abhängigkeit mit der angestrebten Zusammensetzung des gewünschten synthetischen Organextraktes und beziehen sich jeweils auf 1 Liter Endvolumen. Die Angaben beziehen sich demzufolge auf die jeweiligen Endkonzentrationen.

Die für die Herstellung des gewünschten synthetischen Organextrakts erforderliche Teillösung II kann beispielsweise wie folgt zubereitet werden: Ein oder mehrere Peptone, die aus Casein, Soja, Gelatine, Hefe, partiell dehydratisierter Hefe, Lactalbumin oder ähnlichen tierischen bzw. pflanzlichen Ausgangsstoffen gewonnen worden sind, werden zur Desodorierung und Entfärbung in der 10- bis 40-fachen Menge wie destilliertem Wasser unter starkem Rühren gelöst bzw. suspendiert und über Nacht unter Rühren mit Aktivkohle behandelt, und zwar mit einer Menge, die 5 bis 15 Gew.%, bezogen auf die Gesamtmenge der eingesetzten Peptone, entspricht. Nach Abnutschen und Sterilfiltration des Ansatzes durch ein 0,2 $\mu$m Filter (Millipore[R]) wird die Peptonlösung zu der Teillösung I gegeben. Dabei wird die Menge so bemessen, daß die Endlösung 2,0 bis 12,0 g/l Pepton enthält.

Alternativ kann die Teillösung II hergestellt werden, indem man ein oder mehrere tierische Peptone mit der 9- bis 12-fachen Menge 0,5 bis 1,2 N HCl im Rührautoklaven 1 bis 4 Stunden lang auf 100°C erhitzt, anschließend auf 15°C abkühlt und mit einer solchen Menge NaOH versetzt, daß das erhaltene Partialhydrolysat 1 N an NaOH ist. Nach einer Zeitdauer von 90 Minuten wird der pH-Wert der Lösung mit HCl auf 6,8 eingestellt und der Ansatz entsalzt. Anschließend kann die erhaltene Lösung wie oben mit Aktivkohle behandelt und gegebenenfalls nach Sterilfiltration der Teillösung I hinzugefügt werden.

Alternativ können auch geeignete Peptone tierischen Ursprungs, wie insbesondere Kollagenpepton des Typs I mit bakterieller Kollagenase in Tris-HCl-Puffer bei einer Temperatur von 36,8 °C und einem pH-Wert von 7,4 in Gegenwart von $Ca^{++}$-Ionen behandelt werden. Nach einer Inkubationszeit von 5 Stunden wird der Ansatz dialysiert und nach Einstellen des pH-Wertes auf 6,8 und Sterilfiltration der Teillösung I hinzugegeben.

Die Erfindung wird nachfolgend anhand von Beispielen näher erläutert.

## Beispiel 1

## Herstellung eines synthetischen Placentaextraktes

Die nachfolgend aufgeführten Bestandteile wurden unter sterilen Bedingungen und unter Rühren in einem Volumen bidestillierten Wassers gelöst, welches 3/10 bis 4/10 des einzustellenden Endvolumens entspricht. Dabei wurde der pH-Wert der Lösung zwischen 6,0 und 7,5 gehalten.

Die nachfolgend aufgeführten Mengen der dem Ansatz zugegebenen Komponenten beziehen sich jeweils auf 1 l Endvolumen.

(a) Aminosäuren und Derivate

| | |
|---|---|
| L-Glutaminsäure | 0,3 g |
| L-Histidin | 0,06 g |
| L-Ornithin | 0,08 g |
| L-Asparagin | 0,05 g |
| L-Asparaginsäure | 0,15 g |
| L-Valin | 0,05 g |
| L-Tyrosin | 0,03 g |
| DL-Threonin | 0,04 g |
| L-Hydroxyprolin | 0,2 g |
| L-Phenylalanin | 0,04 g |
| Kreatinin | 0,01 g |
| L-Methionin | 0,02 g |
| L-Isoleucin | 0,001 g |
| L-Tryptophan | 0,02 g |
| L-Leucin | 0,05 g |
| β-Alanin | 0,035 g |
| Cystein | 0,002 g |
| L-Alanin | 0,24 g |
| Glycin | 0,4 g |
| L-Prolin | 0,24 g |
| L-Serin | 0,26 g |
| L-Arginin | 0,3 g |
| L-Lysin | 0,3 g |
| Hippursäure | 0,001 g |
| Harnstoff | 0,8 g |

(b) <u>Peptide und Derivate</u>

| Oligopeptide mit 3 bis 10 Aminosäuren u. pflanzl. Peptone bzw. Peptonhydrolysate | 4,0 g |
|---|---|

(c) <u>Nucleinsäurekomponenten und Derivate</u>

| Adenin | 0,02 g |
|---|---|
| Adenosin | 0,02 g |
| Cytidin | 0,04 g |
| Guanin | 0,01 g |
| Cytosin | 0,03 g |
| Guanosin | 0,02 g |
| Hypoxanthin | 0,02 g |
| Inosin | 0,1 g |
| Thymin | 0,04 g |
| Uracil | 0,02 g |
| Uridin | 0,03 g |
| Xanthin | 0,01 g |
| Harnsäure | 0,001 g |
| Orotsäure | 0,001 g |
| cyclisches AMP | 0,04 g |
| Adenosinmonophosphat | 0,01 g |

(d) <u>Kohlenhydrate und Derivate</u>

| Glucose | 0,15 g |
|---|---|
| Sorbit | 2,0 g |
| Mannit | 0,1 g |

(e) <u>Aliphatische Carbonsäuren</u>

| Bernstein-säure | 1,5 g |
|---|---|
| Äpfelsäure | 0,01 g |
| Citronensäure | 0,1 g |

(f) <u>Alkohole</u>

| Ethanol | 2,0 ml |
|---|---|
| Glycerin | 0,4 ml |
| Benzylalkohol | 0,4 ml |

(g) <u>Vitamine</u>

| Thiamindichlorid | 0,002 g |
|---|---|
| Nikotinsäureamid | 0,01 g |
| Calciumpantothenat | 0,002 g |
| myo-Inosit | 0,05 g |
| Pyridoxol · HCl | 0,6 mg |
| Biotin | 0,1 mg |
| Tocopherolsuccinat | 0,002 g |

(h) Mineralsalze und Spurenelemente

| Magnesiumsulfat | 0,05 g |
|---|---|
| Magnesiumaspartat | 0,05 g |
| Zinkacetat | 0,1 mg |
| Kobaltgluconat | 0,005 mg |
| Mangangluconat | 0,01 mg |
| $NaH_2PO_4 \cdot H_2O$ | 0,05 g |

(i) Sonstige Zusätze

| N-Methylglucamin | 0,4 g |
|---|---|
| Natriumlactat | 1,0 g |

(k) Konservierungsstoffe

| p-Hydroxybenzoesäuremethylester-Natriumsalz | 0,7 g |
|---|---|

Zunächst wurden die Aminosäuren L-Glutaminsäure, L-Asparaginsäure, L-Valin, L-Tyrosin, L-Phenylalanin, L-Isoleucin und L-Leucin in 2 N NaOH vorgelöst. Anschließend erfolgte die Zugabe der Carbonsäuren, Zitronensäure, Bernsteinsäure und Äpfelsäure. Nachdem der pH-Wert der Lösung auf 6,4 eingestellt worden war, wurden dem Ansatz die folgenden Aminosäurekomponenten L-Alanin, L-Histidin, Glycin, L-Ornithin, L-Asparagin, DL-Threonin, L-Hydroxyprolin, Kreatinin, L-Methionin, L-Tryotophan, L-Prolin, L-Serin, L-Arginin • HCl, L-Lysin • HCl, Hippursäure und Harnstoff zugegeben. Die Nucleinsäurekomponenten Xanthin und Guanin wurden in 3 N NaOH bzw. 3 N HCl vorgelöst und dann weitgehend neutralisiert.

Der pH-Wert der nach Zugabe der aufgeführten Komponenten erhaltenen Teillösung I wurde auf 6,4 eingestellt. Anschließend erfolgte unter Rühren die Zugabe der Teillösung II, welche wie nachfolgend angegeben hergestellt wurde. Eine aus Soja, Mais und partiell dehydratisierter Hefe im Gewichtsverhältnis von 1:1:10 gewonnene Peptonmenge von insgesamt 4,0 g wurde zur Desodorierung und Entfärbung in einer 40-fachen Menge bidestillierten Wassers unter starken Rühren gelöst und über Nacht unter weiterem Rühren mit 0,6 g frischer Aktivkohle behandelt. Die so erhaltene Peptonlösung wurde nach Abnutschen und Sterilfiltration durch ein 0,2 μm Filter (Millipore) der oben angegebenen Teillösung I zugegeben.

Anschließend wurden dem Ansatz die synthetischen Peptidfragmente Gly-His-Lys, Gly-His-Pro und Glutathion in einer Gesamtkonzentration von 0,1 mg/l zugegeben.

Abschließend wurde das Endvolumen mit bidestilliertem Wasser auf 1 l gebracht und der pH-Wert auf 6,4 eingestellt. Die Abfüllung des erhaltenen synthetischen Placentaextraktes erfolgte durch ein 0,2 μm Millipore-Filter.

Die Analysendaten des erhaltenen synthetischen Placentaextraktes waren wie folgt:

pH-Wert:        6,4
Trockenstoffgehalt:    1,4 Gew.%

| | |
|---|---|
| N-Gehalt nach Kjeldahl: | 0,1% |
| Aminosäuren: | positiv (Ninhydrin) |
| Stoffwechselaktivität: | |

- Atmungssteigerung von Leberhomogenat >1,8
- Gärungssteigerung von Hefe 1,8

| | |
|---|---|
| Chloridgehalt: | <0,2% |
| Sulfat: | 0,05% |
| Hormone: | negativ |
| Schwermetalle: | <20 ppm |
| Sterilität: | absolut keimfrei |
| Toxikologie $DL_{50}$ Maus: | >25 g/kg |
| Dermatologische Prüfung: | ohne Reizwirkung |
| Haltbarkeit: | >3 Jahre. |

Die kosmetische Wirksamkeit des hergestellten synthetischen Placentaextraktes wurde mit Hilfe des Padberg-Testes geprüft. Dabei wurde eine W/O-Creme mit 1% synthetischem Extrakt hergestellt und diese mit einer Creme verglichen, die 1% natürlichen Placentaextrakt enthielt. Beide Cremes wurden mit einer Placebo-Creme ohne jeglichen Zusatz verglichen.

Der Padberg-Test wurde wie folgt durchgeführt:

- Markieren der Teststelle auf der Haut
- Vorbehandlung der Hautstelle mit einer 1%-igen Lösung eines nichtionischen Tensids zur Beeinträchtigung der Haut ("subjektiv rauhe Haut")
- Waschen der Teststelle mit 37°C warmem Wasser
- Abwischen der Teststelle
- Akklimatisieren des Subjektes bei 20°C und einer relativen Feuchte von 60% für eine Zeitdauer von 45 Minuten
- Auftragen von 20 ml einer Flecklösung, die 20% 0,5%-iges Methylenblau und 80% eines 1%-igen nichtionischen Tensids enthält
- 30 Sekunden langes Einwirken der Flecklösung auf die Teststelle
- Trocknen des Farbstoffs, 1 Minute lang
- Entfernen von überschüssigem Farbstoff mit einer 0,2%-igen Lösung des verwendeten nichtionischen Tensids
- 2-maliges, jeweils 60 Sekunden langes Eluieren mit 2 ml-Portionen einer Natriumlauratlösung (2,3% Natriumlaurat, 48,7% reines Wasser, 49% Isopropylalkohol)
- Bestimmung der Absorption des Eluats bei 660 nm mit einem Spektrometer

Die Testbehandlung wurde durchgeführt, indem über einen Zeitraum von 20 Tagen zweimal täglich eine Probe aufgetragen wurde. Dabei wurde sichergestellt, daß die Testpersonen sowohl 3 Tage vor dem Test als auch während der Testpriode kein anderes Kosmetikum verwendet hatten. Am 21. Tag nach Beginn der Behandlung wurden die oben aufgeführten Verfahrensstufen einschließlich der Elution 4 Stunden vor der abschließenden Bewertung durchgeführt.

In der folgenden Tabelle I ist das jeweilige Verhältnis der Absorption von Methylenblau vor und nach dem Auftragen bei jeder Person für die W/O-Cremes in % aufgeführt, wobei die erfindungsgemäß hergestellte W/O-Creme mit 1% synthetischem Placentaextrakt mit einer W/O-Creme mit 1% natürlichem Placentaextrakt und mit einer W/O-Creme als Placebo verglichen wurde. Die absorbierte Menge an Methylenblau verläuft proportional zur Rauhigkeit der Haut und die in der Tabelle aufgeführten Prozentgehalte wurden gemäß der folgenden Gleichung ermittelt:

$$\text{Hautrauhigkeit (\%)} = \frac{\text{Absorption nach Auftragung}}{\text{Absorption von unbehandelter Haut}} \times 100,$$

wobei davon ausgegangen wird, daß eine mit einer Creme behandelten Haut nur eine kleine Menge Methylenblau absorbiert. Der Mittelwert für 12 Versuchspersonen lag für den natürlichen Placentaextrakt etwas höher als für die erfindungsgemäße Formulierung eines synthetischen Placentaextraktes. Der Placebowert war dementsprechend wesentlich höher.

Tabelle I

| Alter der Versuchsperson | W/O-Creme mit 1% Zusatz des synthetischen Placentaextrakts | W/O-Creme mit 1% Zusatz von natürlichem Placentatextrakt | Placebo |
|---|---|---|---|
| 45 | 53,0 | 60,0 | 95,0 |
| 52 | 59,6 | 68,1 | 92,4 |
| 48 | 60,0 | 50,2 | 96,0 |
| 59 | 55,8 | 60,0 | 90,4 |
| 46 | 58,7 | 52,3 | 95,0 |
| 41 | 64,0 | 71,5 | 76,7 |
| 50 | 62,0 | 63,9 | 90,7 |
| 55 | 70,4 | 65,5 | 79,9 |
| 62 | 40,7 | 58,0 | 76,6 |
| 48 | 48,0 | 55,0 | 80,7 |
| 55 | 60,0 | 58,0 | 89,7 |
| 59 | 44,0 | 65,0 | 78,9 |
| | $\overline{56,4}$ | $\overline{60,6}$ | $\overline{86,6}$ |

## Beispiel 2

Zur Herstellung erfindungsgemäßer synthetischer wäßriger Organextrakte wurden die in Tabelle II aufgeführten Substanzen verwendet. Die Herstellung erfolgte unter sterilen Bedingungen ohne Verwendung organischer Extraktionsmittel sowie unter Vermeidung thermischer Belastung der wäßrigen Mischung.

Die Einzelsubstanzen wurden gemäß Beispiel 1 in Form von Teillösungen jeweils zu synthetischen Organextrakten verarbeitet.

## Tabelle II:

| Nr. | % w/w | Substanzbezeichnung |
|---|---|---|
| 1 | 24 - 36 | Panthenol-D |
| 2 | 0,8 - 1,2 | Lactose-Monohydrat |
| 3 | 0,16 - 0,24 | 4-Hydroxybenzoesäuremethylester Na-Salz |
| 4 | 0,03 - 0,05 | Kaliumsorbat |
| 5 | 0,1 - 0,15 | Benzylalkohol |
| 6 | 0,008 - 0,012 | L-Asparagin-$H_2O$ |
| 7 | 0,0016 - 0,0024 | L-Methionin |
| 8 | 2,4 - 3,6 | Glycin |
| 9 | 0,03 - 0,04 | L-Serin |
| 10 | 0,04 - 0,06 | L-Alanin |
| 11 | 0,002 - 0,003 | ß-Alanin |
| 12 | 0,056 - 0,084 | L-Prolin |
| 13 | 0,004 - 0,006 | DL-Threonin |
| 14 | 0,03 - 0,05 | L-Arginin-HCl |
| 15 | 0,06 - 0,08 | Hydroxyprolin |
| 16 | 0,03 - 0,05 | L-Lysin-HCl |
| 17 | 0,008 - 0,012 | L-Ornithin-HCl |
| 18 | 0,009 - 0,013 | Inosin |
| 19 | 0,0016 - 0,0024 | Adenin |
| 20 | 0,0016 - 0,0024 | Adenosin-Monohydrat |
| 21 | 0,0032 - 0,0048 | Cytidin |
| 22 | 0,0024 - 0,0036 | Cytosin |
| 23 | 0,0027 - 0,0041 | Uridin |
| 24 | 0,0013 - 0,0019 | Guanosin |
| 25 | 0,0018 - 0,0026 | Hypoxanthin |
| 26 | 0,0014 - 0,0022 | Uracil |
| 27 | 0,004 - 0,006 | Thymin |
| 28 | 0,00064 - 0,00096 | Guanin gelöst in 3 ml 20 %-iger HCl |
| 29 | 0,0014 - 0,0022 | Xanthin gelöst in 2 ml 10 %-iger NaOH |
| 30 | 0,00032 - 0,00048 | Harnsäure gelöst in 10 %-iger NaOH |
| 31 | 0,12 - 0,18 | D(-)-N-Methylglukamin |
| 32 | 0,16 - 0,24 | Sorbit |

| 33 | 4,0 - 6,0 | D-Mannit |
|---|---|---|
| 34 | 0,08 - 0,12 | Chondriosomenextrakt P2 (aus Hefe) |
| 35 | 0,0032 - 0,0048 | Natrium-L-t-ascorbat |
| 36 | 0,075 - 0,11 | Natriumlactat 50 %-ig |
| 37 | 0,28 - 0,42 | Ethanol absolut |
| 38 | 0,008 - 0,012 | Natriumchlorid |
| 39 | 0,00024 - 0,00036 | Thiamin* HCl |
| 40 | 0,004 - 0,006 | myo-Inosit |
| 41 | 0,0012 - 0,0018 | Nikotinsäureamid |
| 42 | 0,0013 - 0,0019 | Pyridoxolhydrochlorid |
| 43 | 0,0024 - 0,0036 | Kreatinin |
| 44 | 0,02 - 0,03 | Harnstoff |
| 45 | 0,032 - 0,048 | alpha-D-Glucose |
| 46 | 0,0032 - 0,0048 | $NaH_2PO_4 \cdot H_2O$ |
| 47 | 0,16 - 0,24 | Bernsteinsäuredi-Na-Salz-$6H_2O$ |
| 48 | 0,0048 - 0,0072 | L-Valin |
| 49 | 0,0048 - 0,0072 | L-Tyrosin |
| 50 | 0,004 - 0,006 | L-Leucin |
| 51 | 0,0032 - 0,0048 | L-Phenylalanin |
| 52 | 0,021 - 0,031 | L-Asparaginsäure |
| 53 | 0,027 - 0,041 | L-Glutaminsäure |
| 54 | 0,00056 - 0,00084 | Hippursäure |
| 55 | 0,000048 - 0,000072 | Aminobenzoesäure |
| 56 | 0,0027 - 0,0041 | L-Tryptophan |
| 57 | 0,16 - 0,24 | Citronensäuremonohydrat |
| 58 | 0,032 - 0,048 | Bernsteinsäure z.A. |
| 59 | 0,04 - 0,06 | Glycerin 87 % reinst DAB |
| 60 | 0,0000008 - 0,0000012 | Zinkchlorid (gelöst in 1 ml VE-Wasser) |
| 61 | 0,00000016 - 0,00000024 | Kupferacetat Monohydrat (gelöst in 1 ml $H_2O$) |
| 62 | 1,9 - 2,9 | Peptidlösung (Hefebasis) proteinfrei |
| 63 | 0,16 - 0,24 | Hefeextrakt GfN (über Nacht mit |
| 64 | 0,016 - 0,024 | Aktivkohle gerührt und über ein Sterilfilter abgesaugt) |
| 65 | 0,00048 - 0,00072 | Hyaluronsäure |
| 66 | Differenz zu 100 % w/w | VE-Wasser |

Für den so hergestellten Extrakt ergeben sich folgende charakteristische Daten:

Farbe und Aussehen:        schwach gelblich, klar
Löslichkeit:        kaltwasserlöslich, mischbar mit Ethanol (80 %-ig) 1:1

| | |
|---|---|
| Geruch: | sehr schwach artspezifisch |
| Dichte (20°C): | etwa 1,1 g/cm$^3$ |
| pH-Wert: | 6,4 bis 6,5 |
| Brechungsindex n$_D$20: | etwa 1,4 |
| Trockenrückstand: | 31,0 bis 47,5 % |
| Stickstoffgehalt nach Kjeldahl: | 4,6 bis 7,0 % |
| Aminosäuren (Ninhydrin-Nachweis): | positiv |
| Peptide (Nachweis mit Biuret-Reagenz): | positiv |
| mikrobiologische Reinheit: | keimfiltriert |

## Beispiel 3

**Herstellung eines synthetischen Serum-/Placentaextraktes**

Die Herstellung eines synthetischen kombinierten Serum- und Placentaextraktes erfolgte zunächst bis zur Zugabe der Komponente (k) gemäß Beispiel 1 mit der Abweichung, daß die jeweiligen Mengen der Aminosäuren Serin, Prolin, Arginin und Lysin sowie die Menge der unter (f) angegebenen Alkohole um 50% erhöht wurden. Nach Zugabe der Komponente (i) wurde der pH-Wert auf 6,8 eingestellt. Anschließend wurden dem Ansatz die folgenden Peptidkomponenten hinzugegeben:

a) Synthetische Peptidfragmente gemäß Beispiel 1
b) 0,1 mg des Peptidfragments Arg-Gly-Val-Phe-Arg-Arg
c) Eine aus partiell dehydratisierten Hefen und Soja gewonnene Peptonmischung im Gewichtsverhältnis von 5:1 wurde mit der 10-fachen Menge 1 N HCl 3 Stunden lang auf 100°C im Rührautoklaven erhitzt, sodann auf 15°C abgekühlt und mit einer solchen Menge NaOH versetzt, daß das erhaltene Partialhydrolysat 1 N an NaOH war. Nach 90 Minuten wurde der pH-Wert mit HCl auf 6,8 eingestellt und der Ansatz entsalzt. Die anschließende Behandlung mit Aktivkohle sowie die anschließende Sterilfiltration erfolgte gemäß Beispiel 1. Von der aus dem Partialhydrolysat von Peptonen erhaltenen 5%-igen Peptid-Aminosäure-Lösung wurden 20 g zugegeben.

Das erhaltene Lösungsgemisch wurde auf einen pH-Wert von 6,8 eingestellt und mit bidestilliertem Wasser auf das Endvolumen von 1 l verdünnt und erneut durch ein 0,2 μm Millipore-Filter sterilfiltriert.
Die Eigenschaften des erfindungsgemäß hergestellten synthetischen Extraktes waren wie folgt:

| | |
|---|---|
| Konsistenz: | klare wäßrige Flüssigkeit |
| Geruch: | angenehm, frei von organischen Lösungsmitteln |
| Farbe: | schwach gelblich |
| Löslichkeit: | unbegrenzt mit Wasser mischbar, mit Ethanol mischbar im Verhältnis 1 : 1 |
| pH-Wert: | 6,8 |
| Trockenstoffgehalt: | 2,0 Gew.% |
| N-Gehalt nach Kjeldahl | 0,12% |
| Aminosäuren: | positiv (Ninhydrin) |
| Peptide: | positiv (Biuret) |
| Stoffwechselaktivität | |

- Atmungssteigerung von Leberhomogenat >2,5
- Gärungssteigerung von Hefe >2,0

| | |
|---|---|
| Schwermetalle: | <10 ppm |
| Sterilität: | keimfrei |
| Konservierungsmittel: | 4-Hydroxybenzoesäuremethylester |
| Toxikologie: DL$_{50}$ Maus | >25 g/kg |
| Hormone: | negativ |

Zur Demonstration der Wirksamkeit des erfindungsgemäß hergestellten kombinierten synthetischen Placenta-/Serumextraktes wurden Wundheilungsversuche gemäß DE-PS 37 11 054 sowie anschließend Hautspannungsmessungen durchgeführt. Zum Vergleich wurde ein Präparat auf der Basis einer Kombination der beiden natürlichen Organextrakte herangezogen. Die Versuchsergebnisse sind in Tabelle II zusammengestellt.

Tabelle II

| Zeit nach Setzen der Wunde | Versuchsgruppe | Kontrollgruppe | Differenz |
|---|---|---|---|
| a) Kombination der natürlichen Extrakte aus Serum und Placenta | | | |
| 5 Tage | $216 \pm 15$ | $104 \pm 29$ | 113 |
| 12 Tage | $470 \pm 26$ | $402 \pm 24$ | 68 |
| 16 Tage | $809 \pm 49$ | $719 \pm 40$ | 90 |
| 21 Tage | $1627 \pm 56$ | $1412 \pm 60$ | 215 |
| b) Erfindungsgemäßes Präparat nach Beispiel 3 | | | |
| 5 Tage | $250 \pm 17$ | $105 \pm 30$ | 145 |
| 12 Tage | $530 \pm 32$ | $430 \pm 22$ | 100 |
| 16 Tage | $900 \pm 50$ | $740 \pm 33$ | 160 |
| 21 Tage | $1810 \pm 50$ | $1465 \pm 55$ | 345 |

Die in der Tabelle aufgeführten Ergebnisse dokumentieren die überlegene Wirksamkeit des erfindungsgemäßen Präparats. Dieses führte zu jeweils höheren Werten der Hautspannung, als sie durch das Kontrollpräparat erhalten werden konnten.

Erfindungsgemäß hat sich gezeigt, daß ein synthetischer Organextrakt, der das unter b) genannte Peptidfragment Arg-Gly-Val-Phe-Arg-Arg nicht enthält, ähnliche Eigenschaften und Wirksamkeit wie der in diesem Beispiel beschriebene Extrakt aufweist.

Der oben beschriebene synthetische Serum-/Placentaextrakt mit dem unter b) genannten Peptidfragment ist jedoch wegen seiner überlegenen Wirksamkeit bevorzugt.

**Beispiel 4**

**Herstellung eines synthetischen Milzextraktes**

Jeweils 400 l der in den Beispielen 1 und 3 beschriebenen Teillösungen I bzw. II wurden mit einer Peptidlösung kombiniert, welche 1 g synthetische Peptide enthielt, die in Partialhydrolysaten von Milzproteinen und Milzkollagenen nachgewiesen worden waren. Die kombinierte Lösung wurde anschließend mit bidestilliertem Wasser auf das Endvolumen von 1000 l gebracht und nach Einstellung des pH-Wertes auf 6,5 durch ein Membranfilter mit einer Maschenweite von 0,2 μm sterilfiltriert. Die Analysedaten sind wie folgt:

| | |
|---|---|
| pH-Wert: | 6,5 |
| Trockenstoffgehalt: | 2,5 Gew.% |
| Atmungssteigerungsfaktor: (Warburg) | >2,5 |
| Sterilität: | keimfrei |
| Toxikologie $DL_{50}$ Maus: | >25 g/kg |
| Proteine: | negativ |

Die hohe Stoffwechselaktivität der hergestellten synthetischen Extraktlösung konnte am Proliferationsverhalten von Zellkulturen nachgewiesen werden. Dabei wurden sowohl die Vermehrung als auch die Subkultivierung von Fibroblasten in offenen und geschlossenen Kulturgefäßen unter dem Einfluß der gemäß Beispiel 4 hergestellten Lösung beobachtet.

Zur Durchführung des Versuches wurden unter sterilen Bedingungen aus 9 bis 16 Tage lang bebrüteten Hüherembryos Bindegewebe und Knochengewebe sowie Epithelien explantiert und mit der Plasma-Extrakt-Gel-Methode nach Carrel bei einem pH-Wert von 7,2 und einer Temperatur von 37°C im Brutschrank kultiviert. Es wurde die Plasma-Clot-Technik mit Hühnerplasma, Hühner-Embryonalextrakt und Hühnerserum angewendet. Die Testlösungen wurden mit Ringer-Lactatlösung verdünnt. Jeweils 0,02 ml der jeweiligen Konzentrationsstufen wurden dem Kulturmedium zugegeben.

In den folgenden Versuchsreihen wurden Kontrollproben ohne Präparatzusatz zum Vergleich herangezogen.

EP 0 552 516 B1

1) 1 ml der gemäß Beispiel 4 hergestellten Lösung wurde mit 100 ml Ringer-Lactatlösung verdünnt und es wurden jeder Kultur 0,02 ml zugesetzt. Nach einem Zeitraum von 4 Tagen ergaben sich folgende Reaktionen:

(a) Haut:          sehr starke, stimulierte Zellproliferation, bedeutend stärker als bei den Kontrollen.
(b) Herzmuskel:   sehr gute, stimulierte Zellproliferation, stärker als bei den Kontrollen.

2) Bei einer weiteren Verdünnung (1 ml Originallösung/300 ml Ringer-Lactatlösung) zeigten die Explantate des 12 Tage alten Hühnerembryos nach 4 Tagen folgende Resultate:

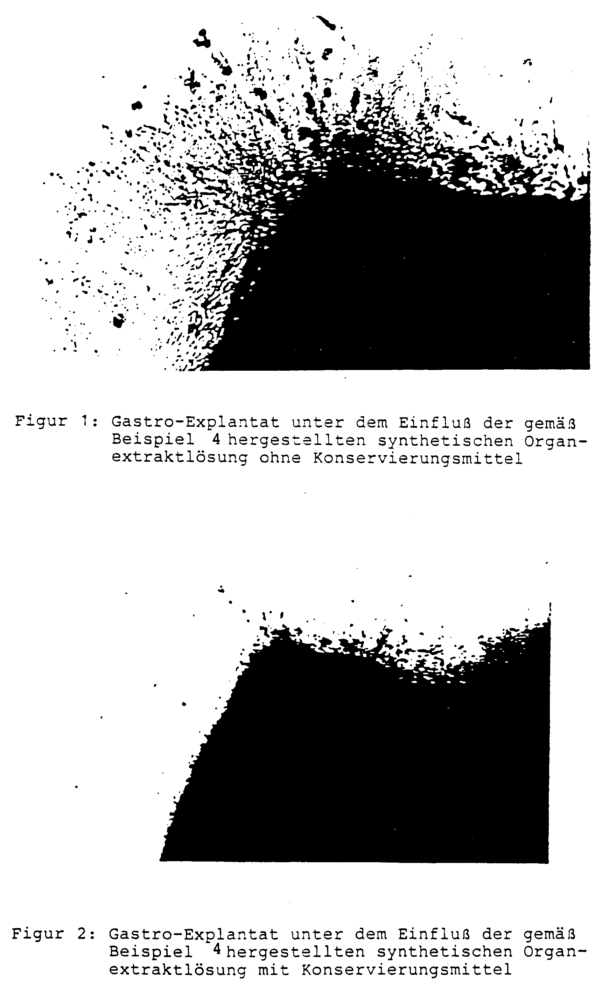

(a) Haut:            sehr gute Proliferation, schwach bei den Kontrollen.
(b) Koronargefäße:   sehr starke, stimulierte Fibroblasten-Proliferation, bedeutend stärker als bei den Kontrollen.
(c) Herzmuskel:      sehr feinfädige, strukturierte Fibroblasten-Proliferation, Kontrolle schlechter.
(d) Leber:           gute Zellproliferation.
(e) Os frontale:     sehr starke Osteoblasten-Proliferation.
(f) Magen:           gute Epithel-Proliferation bei starker Lyse (vgl. Fig. 2).

## Beispiel 5

**Herstellung eines synthetischen Thymusextraktes (100 l-Ansatz)**

200 g p-Hydroxybenzolsäuremethylester in 0,7 l Ethanol (94%-ig) und 0,1 l Benzylalkohol wurden mit bidestilliertem Wasser auf 20 l verdünnt. Hierzu wurden die folgenden Fraktionen in der angegebenen Reihenfolge unter leichtem Rühren zugegeben. Die angegebenen Mengen beziehen sich jeweils auf 1 l der späteren Gesamtlösung.

| | | | |
|---|---|---|---|
| 1) | Glycin | | 0,8 g |
| | L-Alanin, L-Serin, L-Lysin · HCl, L-Prolin, L-Arginin · HCl | | je 2,0 g |
| | D,L-Threonin, L-Histidin, L-Asparagin | | je 0,2 g |
| | L-Methionin | | 0,02 g |
| | Harnstoff | | 2 g |
| | Sorbit | | 50 g |
| 2) | L-Asparaginsäure, L-Leucin, L-Tyrosin, L-Phenylalanin, L-Lysin (vorgelöst in 2 N NaOH) | | je 0,1 g |
| | L-Glutaminsäure, L-Valin | | je 0,6 g |
| 3) | Citronensäure | | 2 g |
| | Äpfelsäure | | 0,1 g |
| | Bernsteinsäure | | 2,5 g |
| 4) | n-Methylglucamin Einstellen des pH-Wertes auf 6,8 | | 1 g |
| 5) | Natriumdihydrogenphosphat | | 0,2 g |
| | Natriumlactatlösung (50%-ig) | | 5 g |
| | Glycerin | | 2 g |
| 6) | Desodorierte Pepton-Lösung, hergestellt aus 1000 g Pepton, welches in Wasser gelöst, mit 700 g Aktivkohle versetzt, 24 Stunden gerührt und filtriert worden war. Insgesamt: | | 25 l |
| 7) | Glucose | | 0,4 g |
| 8) | Inosin | | 0,3 g |
| 9) | Adenin, Adenosin, Guanosin, Thymin, Cytidin, Cytosin, Uridin, Uracil, cycl. AMP, cycl. GMP, ADP, Thiamin-dichlorid | | je 0,01 g |
| 10) | Zinkacetat, Magnesiumacetat, Kobaltgluconat, Mangangluconat | | je 0,003 mg |
| 11) | Chondriosomenextrakt (aus Hefe) | | 2 ml |

Zu dieser Grundlösung, die ein Volumen von etwa 45 l aufwies, wurden die folgenden Peptidlösungen hinzugegeben:

| | |
|---|---|
| - Oligopeptidlösung, enthaltend 0,05 Gew.% der Tri- und Hexapeptide Gly-Pro-His, Gly-His-Lys, Gly-His-His-Gly-His-Lys<br><br>- Thymusfaktor-Fragmente Ala-Lys-Ser-Glu-Gly-Gly-Ser-Asn, Gly-Gly-Glu-Arg-Lys-Asp-Val-Tyr-Val-Glu-Leu-Tyr-Leu, Gly-Gly-Glu-Arg-Lys-Asp-Val-Tyr-Val-Glu-Leu-Tyr-Leu-Val-Tyr-Leu, Gly-Gly-Glu-Arg-Lys-Asp-Val-Tyr-Val-Glu-Leu | 0,1 mg |

Anschließend wurde der pH-Wert der erhaltenen Lösung auf 6,8 eingestellt und der Ansatz mit bidestillilertem Wasser auf das Endvolumen von 100 l aufgefüllt. Danach wurde der pH-Wert der Lösung erneut auf pH 6,8 eingestellt und der Ansatz durch ein 0,2 $\mu$m Millipore-Filter sterilfiltriert.

Der so erhaltene synthetische Thymusextrakt wies die folgenden Eigenschaften auf:

| | |
|---|---|
| Aussehen: | klare Lösung |
| Farbe: | farblos bis schwach gelb |
| Geruch: | desodoriert |
| pH-Wert: | 6,8 |
| Trockenfeststoffgehalt | 5,3 Gew.% |
| Stickstoffgehalt: | 0,7% |
| Aminosäuren: | positiv |
| Peptide: | positiv |
| Stoffwechselaktivität | |
| | - Atmungssteigerungsfaktor: >2,5 |
| Schwermetalle: | <10 ppm |
| Sterilität: | keimfrei |
| Haltbarkeit: | >3 Jahre |

Die toxikologischen und pharmakologischen Daten waren wie folgt:

- Akute Toxizität an Mäusen (80 Tiere)

$DL_{50}$    i.v. >25 ml/kg
        i.p. >50 ml/kg
        p.o. >25 g/kg

- Akute Toxizität an Ratten (80 Tiere)

$DL_{50}$    i.v. >10 ml/kg
        i.p. >20 ml/kg

- Studie der chronischen Toxizität an Albinoratten (40 Tiere)
     keine Toxizität
- Toxizität bei Hunden (12 Beagle-Hunde, 26 Wochen alt)
     keine Toxizität
- Untersuchungen der Reproduktion, Fertilität und Teratogenität bei Ratten (80 Tiere)
     keine Beeinträchtigung
- Toxizitätsstudien, peri- und postnatal an Ratten (30 Tiere, 21 Tage Schwangerschaft, Alter der Tiere ca. 100 Tage)
     keine negativen Befunde
- Untersuchungen teratogener Wirkungen bei Kaninchen (20 Tiere, Körpergewicht Ca. 2,4 kg)
     keine negativen Befunde

Erfindungsgemäß hat sich gezeigt, daß ein in seinen Eigenschaften und seiner Wirkung ähnlicher Thymusextrakt hergestellt werden kann, wenn man anstelle der Tri- und Hexapeptide enthaltenden Oligopeptidlösung nur die genannten Tripeptide, vorzugsweise Gly-His-Lys, verwendet. Auch die alleinige Verwendung des Thymusfaktor-Fragments Ala-Lys-Ser-Glu-Gly-Gly-Ser-Asn anstelle der oben genannten Mischung von Oligopeptiden führt zu akzeptabelen Eigenschaften des synthetischen Thymusextraktes. Die vorstehend erläuterten Modifikationen hinsichtlich der Zusammensetzung der eingesetzten Oligopeptidlösung und der verwendeten Thymusfaktor-Fragmente können entweder getrennt oder zusammen vorgenommen werden; sie liefern einen synthetischen Organextrakt, der ähnlich vorteilhafte Eigenschaften aufweist wie der erfindungsgemäß bevorzugte Thymusextrakt, welcher alle der in diesem Beispiel genannten Einzelbestandteile enthält.

## Beispiel 6

**Herstellung eines synthetischen Serumextraktes (50 l-Ansatz)**

15 g p-Hydroxybenzoesäurealkylester (Natriumsalz) wurden in 20 l bidestillierten Wassers gelöst und es wurden anschließend die folgenden Fraktionen unter Rühren und Stickstoffbegasung hinzugegeben. Die angegebenen Mengen beziehen sich jeweils auf 1 l der späteren Gesamtlösung.

| | | | |
|---|---|---|---|
| 1) | Adenin, Adenosin, Uracil, Uridin, cycl. AMP, cycl. GMP, cycl. TMP, ADP, GDP, Cytosin, Uridin, Cytidin, Thymin, Guanin, Guanosin, ATP-Mg und Hypoxanthin | | je 0,001 g |
| | Inosin | | 0,3 g/l |
| 2) | Glycin | | 1 g |
| | L-Alanin | | 0,2 g |
| | β-Alanin | | 0 1 g |
| | L-Lysin, L-Arginin, L-Prolin, L-Serin | | je 0,3 g |
| | D,L-Threonin | | 0,01 g |
| | L-Leucin, L-Methionin | | je 0,04 g |
| 3) | Glucose | | 0,3 g |
| | Fructose | | 0,02 g |
| 4) | Sorbit | | 9,0 g |
| | Mannit | | 0,5 g |
| | Harnstoff | | 1,5 g |
| 5) | Harnsäure, vorgelöst in heißer 2 N NaOH | | 0,08 g |
| 6) | L-Glutaminsäure | | 0,06 g |
| | L-Asparaginsäure | | 0,05 g |
| | L-Tyrosin | | 0,02 g |
| | L-Phenylalanin | | 0,03 g |
| | L-Valin | | 0,04 g |
| Die vorgenannten Aminosäuren wurden in 2 N NaOH vorgelöst. | | | |
| | Hippursäure | | 0,03 g |
| | p-Aminobenzoesäure | | 0,002 g |
| | Kreatinin | | 0,01 g |
| 7) | Citronensäure | | 2,4 g |
| | Bernsteinsäure | | 1 g |
| | Milchsäure | | 5 g |
| 8) | n-Methylglucamin | | 5 g |
| 9) | Ethanol (95%-ig) | | 5 ml |
| 10) | Natriumchlorid | | 1,0 g |
| | Natriumascorbat | | 0,03 g |
| 11) | Chondriosomenextrakt (aus Hefe) dialysiert, ultrafiltriert und sterilfiltriert, Feststoffgehalt 0,25% | | 3 ml |
| 12) | Spurenelemente gemäß Beispiel 1 und 3 | | |
| 13) | Serumalbumin-Fragmente Arg-Gly, Arg-Gly-Val-Phe-Arg-Arg, Arg-Gly-Val-Phe, Arg-Gly-Val-Phe-Arg | | 0,01 mg |

Die erhaltene Extraktlösung wurde auf einen pH-Wert von 6,7 eingestellt und mit bidestilliertem Wasser auf das Endvolumen von 50 l gebracht. Anschließend wurde der pH-Wert nochmals kontrolliert und der Ansatz durch ein 0,2 μm Millipore-Filter abgefüllt.

Folgende Analysedaten des synthetischen Serumextraktes wurden ermittelt:

26

| pH-Wert: | 6,7 |
| Stickstoffgehalt: | 1,5 mg/ml |
| Trockenfeststoffgehalt: | 2,9 Gew.% |
| Atmungssteigerungsfaktor: | 2,2 |
| Peptidnachweis: | positiv |
| Sterilität: | keimfrei |
| Stabilität: | über 3 Jahre. |

Mit dem auf diese Weise erhaltenen synthetischen Serumextrakt wurde dessen Einfluß auf das Wachstum geschädigter Fibroblasten nach der für Actihämyl beschriebenen Methode von W. Fraefel, Forschungslaboratorien der Solco-Basel durchgeführt. Zur Bestimmung der DNA-Syntheseaktivität wurden die Einbauraten von Thymidin über einen Zeitraum von 6 Tagen an Kulturgruppen mit definierter Zellzahl gemessen:

| Kontrollgruppe I | ungeschädigte Fibroblasten und Epithelzellen |
| Gruppe II | mit $CH_2O$ geschädigte Fibroblasten und Epithelzellen (reversibel geschädigt) |
| Gruppe III | mit $CH_2O$ geschädigte und durch Zusatz von synthetischem Serumextrakt behandelte Fibroblasten und Epithelzellen |
| Gruppe IV | mit $CH_2O$ geschädigte und durch Zusatz eines natürlichen Serumextraktes behandelte Fibroblasten und Epithelzellen |

Die Ergebnisse sind in der nachfolgenden Tabelle III dargestellt.

Tabelle III

| Thymidineinbaurate $1 \times 10^3$/Minuten | | | | |
|---|---|---|---|---|
| Gruppe | Tage 0 | 2 | 4 | 6 |
| I Kontrolle | 0 | 40 | 205 | 400 |
| II | 0 | 8 | 10 | 10 |
| III synt. Serumextrakt | 0 | 18 | 100 | 370 |
| IV | 0 | 16 | 110 | 360 |

Die angegebenen Werte repräsentieren Mittelwerte aus jeweils 10 Versuchen. Die wiedergegebenen Ergebnisse zeigen deutlich, daß die durch $CH_2O$ geschädigte Fibroblastenkultur sowohl mit dem natürlichen wie auch dem erfindungsgemäßen synthetischen Serumextrakt hinsichtlich ihrer DNA-Syntheserate der als Kontrolle dienenden Kulturgruppe I angepaßt werden konnte.

Bei der ausschließlichen Verwendung des leicht erhältlichen Dipeptids Arg-Gly anstelle aller der oben unter 13) genannten Serumalbumin-Fragmente kann ein synthetischer Serumextrakt erhalten werden, dessen Wirksamkeit derjenigen des oben beschriebenen Extrakts ähnlich ist. Die Verwendung aller unter 13) genannten Serumalbumin-Fragmente führt jedoch zur vollen Entfaltung der oben im Vergleich zum natürlichen Serumextrakt beschriebenen Ergebnisse und ist erfindungsgemäß bevorzugt.

**Beispiel 7**

**Synthetischer Kollagenextrakt**

Eine Analyse der Aminosäurezusammensetzung eines Kollagenhydrolysats ergab folgendes Aminosäure-Spektrum:

| Aminosäure-Septrum | |
|---|---|
| | g AS/100 g Protein |
| Alanin | 10,2 |
| Arginin | 8,8 |
| Asparaginsäure | 7,0 |
| Glutaminsäure | 12,0 |
| Glycin | 26,5 |
| Histidin | 1,5 |
| Hydroxylysin | 1,1 |
| Hydroxyprolin | 12,5 |
| Isoleucin | 1,8 |
| Leucin | 4,0 |
| Lysin | 4,4 |
| Methionin | 0,5 |
| Phenylalanin | 2,2 |
| Prolin | 16,5 |
| Serin | 3,7 |
| Threonin | 2,4 |
| Tyrosin | 0,5 |
| Valin | 2,9 |

Es wurden 100 g der analytisch gefundenen Aminosäuren in ihren entsprechenden Gewichtsverhältnissen in 5 l bidestilliertem Wasser gelöst. Anschließend wurden dieser Lösung jeweils 10 g der Aminosäuren Glycin, L-Prolin, L-Hydroxyprolin, L-Arginin, L-Lysin und L-Serin zugegeben. Diese Lösung wurde insgesamt viermal hergestellt und mit jeweils einer der nachfolgend aufgeführten Peptidlösungen vereinigt und anschließend mit bidestilliertem Wasser auf ein jeweiliges Endvolumen von 6 l gebracht.

Peptidlösungen:

a) 30 g Pepton wurden in 900 ml Wasser gelöst und mit 10 g Aktivkohle behandelt, über Nacht gerührt und dann filtriert.

b) Peptidlösung, erhalten durch eine 6 Stunden lange Partialhydrolyse eines Peptons mit 2,5 N HCl im Rührautoklaven bei einer Temperatur von 110°C. Die Lösung wurde nach Abkühlen und Filtrieren durch Zugabe von NaOH auf eine Alkalität von 1 N NaOH gebracht und durch Dialyse entsalzt. Nach einer 24 Stunden langen Behandlung mit Aktivkohle wurde der pH-Wert der Lösung auf 6,0 eingestellt und sterilfiltriert, bevor das Partialhydrolysat aus Peptiden und Aminosäuren der Hauptlösung in einer Menge von 5 Gew.% zugegeben wurde.

c) Eine durch Partialhydrolyse von Peptonen mittels geeigneter Proteinasen hergestellte Peptidlösung wurde nach Dialyse mit NaOH auf einen pH-Wert von 8,5 eingestellt und 4 Tage lang bei einer Temperatur von 6°C gelagert. Anschließend wurde die Lösung auf eine Alkalinität von 1 N NaOH gebracht und nach 90 Minuten auf einen pH-Wert von 6,4 eingestellt. Im Anschluß an eine Behandlung mit Aktivkohle, einer Entsalzung und einer Filtration wurde eine Peptidaminosäurelösung erhalten, die sterilfiltriert und der Grundlösung in einer Menge von 5 Gew.% zugegeben wurde.

d) Unter Verwendung der Tripeptide Glutathion, Gly-Pro-His, Gly-Ala-Pro, Gly-His-Lys, Gly-Hyp-Pro, Gly-Pro-Ala,

EP 0 552 516 B1

Gly-Lys-Pro, Gly-His-Arg, Gly-Lys-Lys, Gly-Lys-His, Gly-Gly-His, Gly-Arg-His und Gly-His-Hyp in einer Gesamtmenge von 1000 mg in bidestilliertem Wasser wurde eine synthetische Peptidlösung hergestellt, die sterilfiltriert und der Hauptlösung zugegeben wurde.

Den vier auf diese Weise erhaltenen Ansätzen von jeweils 6 l wurden dann die in Beispiel 1 unter (c) bis (g) und (i) genannten Komponenten zugegeben. Die Ansätze wurden anschließend getrennt ultrafiltriert (Molgewicht unter 3000) und durch ein Millipore-Filter mit einer Maschenweite von 0,2 μm sterilfiltriert und in Vorratsgefäße abgefüllt.

Es zeigte sich, daß die Hautpflegeeigenschaften und die Eigenschaften der Zellaktivität und des Feuchtigkeitsgehaltes von Creme-Grundlagen unter Verwendung von 2 bis 5 Gew.% dieser synthetischen Kollagenextrakte, bezogen auf das Gesamtgewicht der Salbengrundlage, eindeutig verbessert werden konnten. Es wurde eine Reihe von Creme-Grundlagen hergestellt, welche die folgenden Gewichtsteile enthielten:

| Cetanol | 41 Gew.% |
|---|---|
| Vaseline | 14 Gew.% |
| Polyoxyethylenlaurylether | 0,4 Gew.% |
| Sorbitansequioleat | 4 Gew.% |
| synthetischer Kollagenextrakt | 4 Gew.% |
| Wasser          ad | 100 Gew.% |

Zum Nachweis der gesteigerten Zellaktivität wurden Zellkulturtests, Warburg-Versuche zur Zellatmung sowie Versuche zur Steigerung des Wachstums von Fibroblasten (vgl. Beispiel 6) durchgeführt.

Ergebnisse:

| Zellkulturtest: Klontest gemäß Beispiel 9 | 190% |
|---|---|
| Atmungssteigerungsfaktor (Warburg): | 2,2% |
| Fibroblastenwachstum nach 6 Tagen (Thymidin-Einbaurate): | $350 \times 10^3$ |

Erfindungsgemäß hat sich gezeigt, daß ein synthetischer Kollagenextrakt, der nur die unter d) genannten Tripeptide Glutathion, Gly-His-Lys und Gly-His-Arg, vorzugsweise Gly-His-Lys und Gly-His-Arg, sowohl einzeln als auch in Kombination enthält, in seiner Wirksamkeit und seinen Eigenschaften dem in diesem Beispiel beschriebenen synthetischen Kollagenextrakt ähnlich ist.

Der optimale Effekt wird jedoch durch die Verwendung aller unter d) genannten Tripeptide erzielt und ist daher bevorzugt.

## Beispiel 8

**Synthetischer Bindegewebsextrakt**

In einer wäßrigen Lösung, die 0,1% 4-Hydroxybenzoesäureester und 0,2% sorbinsaures Kaliumsalz enthielt, wurden die folgenden Bestandteile in der angegebenen Reihenfolge gelöst, wobei sich die Mengen auf jeweils 1 l des Gesamtansatzes beziehen:

| a) | Glycin, Alanin, Prolin, Hydroxyprolin | je 0,6 g |
|---|---|---|
| | Asparaginsäure, Serin, Glutaminsäure, Lysin, Arginin | je 0,3 g |
| | Threonin, Valin, Methionin, Isoleucin, Leucin, Phenylalanin, Histidin, Tyrosin | je 0,08 g |
| b) | Peptide, die durch chemische Hydrolyse (NaOH, HCl) von Gelatinepeptonen und 90 Minuten langes Nachbehandeln mit 1 N NaOH, Entsalzung und ultrafiltration (Molgewicht unter 2000) erhalten wurden | 10 g |
| c) | Synthetische Peptidfragmente Glutathion, Gly-Pro-His, Gly-Ala-Pro, Gly-His-Lys, Gly-Hyp-Pro, Gly-Pro-Ala, Gly-Lys-Pro, Gly-His-Arg, Gly-Lys-Lys, Gly-Lys-His, Gly-Gly-His, Gly-Arg-His und Gly-His-Hyp | 1g |
| d) | Komponenten (c), (d), (e), (f), (g) und (i) gemäß Beispiel 1. | |

Der auf diese erhaltene Ansatz wurde durch ein Millipore-Filter mit einer Maschenweite von 0,2 µm sterilfiltriert und es wurden unter sterilen Bedingungen die folgenden hochmolekularen Bindegewebskomponenten hinzugegeben:

| | |
|---|---|
| Hyaluronsaures Natrium | 2 g/l |
| Chondroitinsulfat (Natriumsalz) | 17,5 g/l |
| Keratinsulfat | 1 g/l |

Die genannten hochmolekularen Komponenten wurden vor Zugabe zu dem Ansatz durch mehrfache Begasung mit Ethylenoxid und Nachspülen mit Stickstoff keimfrei gemacht. Diese Maßnahme ist erforderlich, um eine sterile Endlösung zu erhalten, da die Endlösung aufgrund ihres Gehaltes an hochmolekularen Komponenten nicht sterilisiert werden kann.

Bei der Analyse der synthetischen Bindegewebsextraktlösung wurde die folgenden Daten ermittelt:

| | |
|---|---|
| pH-Wert | 6,0 |
| Trockenstoffgehalt | 6% |
| Stickstoffgehalt | 0,9% |
| Mucopolysaccharide | 1,1% |
| Schwermetalle unter | 20 ppm |
| Sterilität | keimfrei |

Ferner hat sich gezeigt, daß ein synthetischer Bindegewebsextrakt, der nur die unter c) genannten Tripeptide Glutathion und Gly-His-Lys, vorzugsweise Gly-His-Lys enthält, in seiner Wirksamkeit und seinen Eigenschaften dem in diesem Beispiel beschriebenen synthetischen Kollagenextrakt ähnlich ist. Zur vollen Wirksamkeit tragen jedoch alle die unter c) genannten Tripeptide bei und ein derartiger Extrakt ist daher bevorzugt.

## Beispiel 9

## Synthetischer Organextrakt ohne Konservierungsmittel

Die Herstellung von synthetischen Organextrakten erfolgte gemäß vorstehender Beispiele, jedoch ohne Zusatz von Konservierungsmitteln und ohne Zusatz von Alkoholen. Überraschend ist jedoch, daß die auf diese Weise hergestellten synthetischen Organextrakte immer noch eine Haltbarkeit bzw. Stabilität von mehr als 2 Jahren aufwiesen. Insbesondere zeigten solche Präparate bei der Anwendung als kosmetische oder medizinische Additive eine stärkere Aktivität auf das Wachstum von Zellkulturen als die Konservierungsmittel enthaltenden Additive der genannten Beispiele:

I. Bei dem gemäß Beispiel 4 beschriebenen synthetischen Milzextrakt ohne konservierende Zusätze wurde unter Anwendung der im Beispiel 4 beschriebenen Methode eine starke Stimulanz auf die Zellproliferation ermittelt, wie in Fig. 1 zu sehen ist. Die Fig. 2 zeigt im Vergleich dazu ein Explantat unter Einwirkung des gemäß Beispiel 4 hergestellten Additivs mit Konservierungszusätzen.

II. Mit Hilfe des Klontestes wurden die gemäß Beispiel 1, 3 und 6 hergestellten synthetischen Präparate mit und ohne Zusatz von Konservierungsmitteln und Zusätzen getestet:

Zur Durchführung des Klontests wurden Baby-Hamster-Kidney-Zellen (BHK-Zellen) in einer Zelldichte von 100 Zellen/Petrischale ausgesät. Das eingesetzte Nährmedium enthielt D-MEM + 1% NEAA + 1% Glutamin sowie fötales Kälberserum (10%-ig) und wurde den Petrischalen in einem Gesamtvolumen von jeweils 5 ml zugegeben. Die Inkubationszeit betrug 6 Tage bei einer Temperatur von 37°C und einer $CO_2$-Atmosphäre von 6%. Die Ergebnisse des Klontests sind in der Tabelle 4 zusammengestellt.

Tabelle IV

| Präparat aus Beispiel | Klontest ohne | Klontest mit |
|---|---|---|
| | Konservierungsmittel | |
| 1 | 177 % | 120 % |
| 3 | 210 % | 140 % |
| 6 | 165 % | 120 % |
| Kontrolle | 100 % | 100 % |

**Patentansprüche**

1.  Synthetischer wäßriger Organextrakt, der mindestens

    (a) eine Aminosäurekomponente mit monomeren Aminosäuren oder Aminosäurederivaten,
    (b) eine Peptidkomponente,
    (c) eine Nucleobase, eine Nucleosid-, Nucleotid- oder Nucleinsäurekomponente,
    (d) eine Kohlenhydratkomponente und/oder eine Kohlenhydratderivatkomponente,
    (e) eine aliphatische Carbonsäure oder deren Salz mit 3 bis 6 Kohlenstoffatomen,
    (f) einen aliphatischen und/oder aromatischen Alkohol mit 2 bis 7 Kohlenstoffatomen,
    sowie gegebenenfalls
    (g) Vitamine,
    (h) Mineralsalze und/oder Spurenelemente,
    (i) Puffersubstanzen und
    (k) Konservierungsstoffe

    enthält, **dadurch gekennzeichnet,** daß die Aminosäurekomponente (a) und die Peptidkomponente (b) eine oder mehrere der zu den Gruppen

    (I) Glycin, L-Prolin, L-Hydroxyprolin, L-Alanin,
    (II) L-Glutaminsäure, L-Asparaginsäure, L-Asparagin,
    (III) L-Arginin, L-Serin, L-Lysin

    gehörenden Aminosäuren enthält, wobei sich der Extrakt zu mindestens

    -   0,20 Gew.% aus Aminosäure(n) der Gruppe (I),

31

- 0,05 Gew.% aus Aminosäure(n) der Gruppe (II) und
- 0,05 Gew.% aus Aminosäure(n) der Gruppe (III)

zusammensetzt, und

- die Komponente (c) mit einem Anteil von mindestens 0,02 Gew.%,
- die Komponente (d), die mindestens einen Zuckeralkohol aus der Gruppe bestehend aus Sorbit, Mannit, Inosit und Dulcit umfaßt, mit einem Anteil von mindestens 0,2 Gew.%,
- die Komponente (e) mit einem Anteil von mindestens 0,2 Gew.%, und
- die Komponente (f) mit einem Anteil von mindestens 0,3 Gew.%,

jeweils bezogen auf den Gesamtextrakt, in der synthetischen Extraktlösung enthalten ist.

2. Synthetischer wäßriger Organextrakt nach Anspruch 1, **dadurch gekennzeichnet,** daß die Aminosäurekomponente (a) und die Peptidkomponente (b)

- 2 bis 6 Gew.% Aminosäuren der Gruppe (I),
- 0,05 bis 0,15 Gew.% Aminosäuren der Gruppe (II) und
- 0,10 bis 0,20 Gew.% Aminosäuren der Gruppe (III)

umfassen und

- die Komponente (c) mit einem Anteil von 0,02 bis 0,06 Gew.%,
- die Komponente (d) mit einem Gesamtanteil an Sorbit, Mannit und Inosit von 3 bis 8 Gew.%,
- die Komponente (e) mit einem Anteil von 0,2 bis 0,7 Gew.% und
- die Komponente (f) mit einem Anteil von 0,3 bis 0,7 Gew.%

in der synthetischen Extraktlösung enthalten ist.

3. Synthetischer wäßriger Organextrakt nach den Ansprüchen 1 und 2, **dadurch gekennzeichnet,** daß die Gruppe (I) Glycin mit einem Anteil von 1,5 bis 4,5 Gew.%, vorzugsweise 2,4 bis 3,6 Gew.%, enthält.

4. Synthetischer wäßriger Organextrakt nach den Ansprüchen 1 bis 3, **dadurch gekennzeichnet,** daß die Komponente (d) D-Mannit mit einem Anteil von 3 bis 7 Gew.%, vorzugsweise 4 bis 6 Gew.%, enthält.

5. Synthetischer wäßriger Organextrakt nach den Ansprüchen 1 bis 4, **dadurch gekennzeichnet,** daß als Komponente (g) mindestens D-Panthenol mit einem Anteil von 15 bis 50 Gew.%, vorzugsweise 24 bis 36 Gew.% in der synthetischen Extraktlösung enthalten ist.

6. Synthetischer wäßriger Organextrakt nach Anspruch 5, **dadurch gekennzeichnet,** daß er zusätzlich 0,8 bis 1,2 Gew.% D-Lactose-Monohydrat enthält.

7. Synthetischer wäßriger Organextrakt nach Anspruch 1, **dadurch gekennzeichnet**, daß die Aminosäurekomponente (a) und die Peptidkomponente (b) etwa

- 0,2 bis 0,7 Gew.% Aminosäuren der Gruppe (I),
- 0,05 bis 0,15 Gew.% Aminosäuren der Gruppe (II) und
- 0,10 bis 0,20 Gew.% Aminosäuren der Gruppe (III)

umfassen und

- die Komponente (c) mit einem Anteil von 0,02 bis 0,06 Gew.%,
- die Komponente (d) mit einem Gesamtanteil an Sorbit, Mannit und Inosit von 0,2 bis 0,5 Gew.%,
- die Komponente (e) mit einem Anteil von 0,2 bis 0,7 Gew.% und
- die Komponente (f) mit einem Anteil von 0,3 bis 0,7 Gew.%,

jeweils bezogen auf den Gesamtextrakt,in der synthetischen Extraktlösung enthalten ist.

8. Synthetischer wäßriger Organextrakt nach den Ansprüchen 1 bis 7, **dadurch gekennzeichnet**, daß er Äpfelsäure und/oder Bernsteinsäure mit einem Anteil von 0,02 bis 1,5 Gew.% enthält.

9. Synthetischer wäßriger Organextrakt nach den Ansprüchen 1 bis 8, **dadurch gekennzeichnet,** daß die Komponente (b) Peptide aufweist, die mindestens eine der Aminosäuren ausgewählt aus Serin, Arginin, Lysin, Prolin und Hydroxyprolin enthalten.

10. Synthetischer wäßriger Organextrakt nach den Ansprüchen 1 bis 9, **dadurch gekennzeichnet,** daß die Komponente (b) Peptide aufweist, die mindestens zwei der Aminosäuren Serin, Arginin, Lysin, Prolin, Hydroxyprolin und Histidin, enthalten.

11. Synthetischer wäßriger Organextrakt nach den Ansprüchen 1 bis 10, **dadurch gekennzeichnet,** daß die Komponente (b) im wesentlichen Tri- bis Hexapeptide, Salze derselben und/oder Metallkomplexe derselben aufweist.

12. Synthetischer wäßriger Organextrakt nach den Ansprüchen 1 bis 11, **dadurch gekennzeichnet,** daß sich die Komponente (b) mindestens teilweise aus Peptonen zusammensetzt, welche aus der Gruppe bestehend aus Sojabohnenpepton, Maispepton, Haferpepton, Hefepeptiden und/oder von partiell dehydratisierter Hefe ausgewählt sind.

13. Synthetischer wäßriger Organextrakt nach den Ansprüchen 1 bis 12, **dadurch gekennzeichnet,** daß er monomere Aminosäuren aus der Gruppe bestehend aus Glycin, Prolin, Serin, Lysin, Arginin und Hydroxyprolin mit einem Anteil von 0,05 bis 5,0 Gew.% enthält.

14. Synthetischer wäßriger Organextrakt nach den Ansprüchen 1 bis 13, **dadurch gekennzeichnet**, daß er Hexite in einer Menge von 0,2 bis 8 Gew.% enthält.

15. Synthetischer wäßriger Organextrakt nach den Ansprüchen 1 bis 14, **dadurch gekennzeichnet,** daß die Alkohol-Komponente (f) Benzylalkohol, Glycerin und/oder Ethanol umfaßt.

16. Synthetischer wäßriger Organextrakt nach den Ansprüchen 1 bis 15, **dadurch gekennzeichnet,** daß die Komponente (c) Inosin, Adenin, Adenosin, Guanosin, cycl. AMP und/oder AMP umfaßt.

17. Synthetischer wäßriger Organextrakt nach den Ansprüchen 1 bis 16, **dadurch gekennzeichnet,** daß er frei von Konservierungsmitteln ist.

18. Synthetischer wäßriger Organextrakt nach den Ansprüchen 1 bis 17, **dadurch gekennzeichnet,** daß der Trockenstoffgehalt der synthetischen Organextraktlösung 0,2 - 60 Gew.% beträgt.

19. Verwendung des synthetischen wäßrigen Organextraktes nach den Ansprüchen 1 bis 18 als Ersatz oder als Ergänzung für Placenta-, Thymus-, Blut-, Milz-, Leber-, Kollagen-, Bindegewebs-, Amnionflüssigkeits-, Quallen-, Rogen- und Euterextrakte bzw. für deren Kombinationen.

20. Verwendung des synthetischen wäßrigen Organextraktes nach den Ansprüchen 1 bis 18 zur Herstellung einer kosmetischen Formulierung.

21. Verwendung einer Kombination aus zwei oder mehreren synthetischen wäßrigen Organextrakten nach den Ansprüchen 1 bis 18 zur Herstellung einer kosmetischen Formulierung.

22. Verwendung des synthetischen wäßrigen Organextraktes nach den Ansprüchen 1 bis 18 zur Herstellung eines medizinischen Präparates zur topischen, subkutanen oder intramuskulären Applikation für die Zwecke der äußeren oder inneren Wundheilung.

23. Verwendung des synthetischen wäßrigen Organextraktes nach den Ansprüchen 1 bis 18 zur Herstellung eines medizinisches Präparates zur Stärkung des Immunsystems.

24. Verwendung des synthetischen wäßrigen Organextraktes nach den Ansprüchen 1 bis 18 zur Herstellung eines medizinischen Präparates zur Aktivierung des Zellstoffwechsels.

25. Verwendung des synthetischen wäßrigen Organextraktes nach den Ansprüchen 1 bis 18 zur Herstellung eines medizinischen Präparates zur Behandlung gastroenterologischer Erkrankungen, insbesondere Ulcera.

**Claims**

1. An aqueous synthetic organ-extract containing at least

   (a) an aminoacid component comprising monomeric aminoacids or aminoacid derivatives,
   (b) a peptide component,
   (c) a nucleobase, a nucleoside-, nucleotide- or nucleic acid component,
   (d) a carbohydrate component and/or a carbohydrate derivative component,
   (e) an aliphatic carboxylic acid or salt thereof containing 3 to 6 carbon atoms,
   (f) an aliphatic and/or aromatic alcohol containing 2 to 7 carbon atoms,
   and optionally
   (g) vitamines,
   (h) mineral salts and/or trace elements,
   (i) buffer substances and
   (k) preservatives,

   characterized in that the aminoacid component (a) and the peptide component (b) contain one or more aminoacids belonging to the following groups

   (I) glycine, L-proline, L-hydroxyproline, L-alanine,
   (II) L-glutamic acid, L-aspartic acid, L-asparagine,
   (III) L-arginine, L-serine, L-lysine

   wherein the extract is composed at least of

   - 0.20 weight% of aminoacid(s) of the group (I),
   - 0.05 weight% of aminoacid(s) of the group (II) and
   - 0.05 weight% of aminoacid(s) of the group (III)

   and the synthetic extract solution contains, each based on the total extract,

   - component (c) in a proportion of at least 0.02 weight%,
   - component (d) comprising at least a suger alcohol selected from the group consisting of sorbitol, mannitol, inositol and dulcitol, in a proportion of at least 0.2 weight%,
   - component (e) in a proportion of at least 0.2 weight%, and
   - component (f) in a proportion of at least 0.3 weight%.

2. The aqueous synthetic organ-extract according to claim 1, characterized in that the aminoacid component (a) and the peptide component (b) comprise

   - 2 to 6 weight% of aminoacids of the group (I),
   - 0.05 to 0.15 weight% of aminoacids of the group (II) and
   - 0.10 to 0.20 weight% of aminoacid(s) of the group (III),

   and the synthetic extract solution contains

   - component (c) in a proportion of from 0.02 to 0.06 weight%,
   - component (d) comprising sorbitol, mannitol and inositol in a total proportion of from 3 to 8 weight%,
   - component (e) in a proportion of from 0.2 to 0.7 weight% and
   - component (f) in a proportion of from 0.3 to 0.7 weight%.

3. The aqueous synthetic organ-extract according to claims 1 and 2, characterized in that the group (I) contains glycine in a proportion of from 1.5 to 4.5 weight%, preferably from 2.4 to 3.6 weight%.

4. The aqueous synthetic organ-extract according to any of claims 1 to 3, characterized in that component (d) contains D-mannitol in a proportion of from 3 to 7 weight%, preferably from 4 to 6 weight%.

5. The aqueous synthetic organ-extract according to any of claims 1 to 4, characterized in that the synthetic extract solution contains at least D-panthenol in a proportion of from 15 to 50 weight%, preferably from 24 to 36 weight%, as component (g).

6. The aqueous synthetic organ-extract according to claim 5, characterized in that it additionally contains from 0.8 to 1.2 weight% of D-lactose monohydrate.

7. The aqueous synthetic organ-extract according to claim 1, characterized in that the aminoacid component (a) and the peptide component (b) comprise about

   - 0.2 to 0.7 weight% of aminoacid(s) of the group (I),
   - 0.05 to 0.15 weight% of aminoacid(s) of the group (II) and
   - 0.10 to 0.20 weight% of aminoacid(s) of the group (III)

   and the synthetic extract solution contains, each based on the total extract,

   - component (c) in a proportion of from 0.02 to 0.06 weight%,
   - component (d) comprising sorbitol, mannitol and inositol in a proportion of from 0.2 to 0.5 weight%,
   - component (e) in a proportion of from 0.2 to 0.7 weight% and
   - component (f) in a proportion of from 0.3 to 0.7 weight%.

8. The aqueous synthetic organ-extract according to any of claims 1 to 7, characterized in that it contains malic acid and/or succinic acid in a proportion of from 0.02 to 1.5 weight%.

9. The aqueous synthetic organ-extract according to any of claims 1 to 8, characterized in that component (b) comprises peptides containing at least one of the aminoacids selected from the group consisting of serine, arginine, lysine, proline and hydroxyproline.

10. The aqueous synthetic organ-extract according to any of claims 1 to 9, characterized in that component (b) comprises peptides containing at least two of the aminoacid, serine, arginine, lysine, proline, hydroxyproline and histidine.

11. The aqueous synthetic organ-extract according to any of claims 1 to 10, characterized in that component (b) essentially comprises tri-to hexapeptides, salts thereof and/or metal complexes thereof.

12. The aqueous synthetic organ-extract according to any of claims 1 to 11, characterized in that component (b) at least patially is composed, of peptones selected from the group consisting of soja bean peptone, corn peptone, oat peptone, yeast peptides and/or partially dehydrated yeast.

13. The aqueous synthetic organ-extract according to any of claims 1 to 12, characterized in that it contains monomeric aminoacids selected from the group consisting of glycine, proline, serine, lysine, arginine and hydroxyproline in a proportion of from 0.05 to 5.0 weight%.

14. The aqueous synthetic organ-extract according to any of claims 1 to 13, characterized in that it contains hexitols in an amount of from 0.2 to 8 weight%.

15. The aqueous synthetic organ-extract according to any of claims 1 to 14, characterized in that the alcohol component (f) comprises benzylalcohol, glycerine and/or ethanol.

16. The aqueous synthetic organ-extract according to any of claims 1 to 15, characterized in that component (c) comprises inosine, adenine, adenosine, guanosine, cyclic AMP and/or AMP.

17. The aqueous synthetic organ-extract according to any of claims 1 to 16, characterized in that it is free of preservatives.

18. The aqueous synthetic organ-extract according to any of claims 1 to 17, characterized in that the dry solid contet of the synthetic organ-extract solution is 0.2 to 60 weight%.

19. The use of the aqueous synthetic organ-extract according to any of claims 1 to 18 as a substitute or complement for placenta, thymus, blood, rate, liver, collagen, connective tissue, amnion fluid, jellyfish, roe and udder extracts and combinations thereof.

20. The use of the aqueous synthetic organ-extract according to any of claims 1 to 18 for the preparation of a cosmetic formulation.

21. The use of the combination of two or more aqueous synthetic organ-extracts according to any of claims 1 to 18 for the preparation of a cosmetic formulation.

22. The use of the aqueous synthetic organ-extract according to any of claims 1 to 18 for the preparation of a medical preparation for topic, subcutaneous or intramuscular application for the purpose of external or internal wound healing.

23. The use of the aqueous synthetic organ-extract according to any of claims 1 to 18 for the preparation of a medical preparation for enhancing the immune system.

24. The use of the aqueous synthetic organ-extract according to any of claims 1 to 18 for the preparation of a medical preparation for activating the cell metabolism.

25. The use of the aqueous synthetic organ-extract according to any of claims 1 to 18 for the preparation of a medical preparation for treating gastro-enterologic diseases, in particular ulcera.

**Revendications**

1. Extrait aqueux synthetique d'un organe comprenant au moins

   (a) un composant aminoacidique avec des aminoacides monomères ou des dérivés d'aminoacides,
   (b) un composant peptidique,
   (c) une base nucléonique, un composant nucléosidique, nucléotidique ou d'acide nucléinique,
   (d) un composant glucidique et/ou un composant dérivé de glucide,
   (e) un acide carbonique aliphatique ou son sel avec 3 à 6 atomes de carbone,
   (f) un alcool aliphatique et/ou aromatique avec 2 à 7 atomes de carbone ainsi que, le cas échéant,

   (g) des vitamines,
   (h) des sels minéraux et/ou des oligo-éléments,
   (i) des substances tampon et
   (k) des agents conservateurs,

   caractérisé par le fait que le composant aminoacidique (a) et le composant peptidique (b) comprennent l'un ou plusieurs des aminoacides appartenant aux groupes

   (I) glycine, L-proline, L-hydroxyproline, L-alanine,
   (II) L-acide glutamique, L-acide asparaginique, L-asparagine,
   (III) L-arginine, L-sérine, L-lysine

   l'extrait étant composé d'au moins

   - 0,20% en poids d'aminoacide(s) du groupe (I),
   - 0,05% en poids d'aminoacide(s) du groupe (II) et
   - 0,05% en poids d'aminoacide(s) du groupe (III),

   et

   - le composant (c) avec une quote-part d'au moins 0,02% en poids,
   - le composant (d), qui comprend au moins un alcool de sucre du groupe comprenant le sorbite, le mannitol, l'inosite et le dulcite, avec une quote-part d'au moins 0,2% en poids,
   - le composant (e) avec une quote-part d'au moins 0,2% en poids, et
   - le composant (f) avec une quote-part d'au moins 0,3% en poids,

   respectivement par rapport à l'extrait total, étant contenu dans la solution d'extrait synthétique.

2. Extrait aqueux synthétique d'un organe selon la revendication 1, caractérisé par le fait que le composant aminoacidique (a) et le composant peptidique (b) comprennent

- 2 à 6% en poids d'aminoacides du groupe (I),
- 0,05 à 0,15% en poids d'aminoacides du groupe (II) et
- 0,10 à 0,20% en poids d'aminoacides du groupe (III)

et

- le composant (c) avec une quote-part de 0,02 à 0,06% en poids,
- le composant (d) avec une quote-part totale en sorbite, mannitol et inosite de 3 à 8% en poids,
- le composant (e) avec une quote-part de 0,2 à 0,7% en poids,
- le composant (f) avec une quote-part de 0,3 à 0,7% en poids,

étant compris dans la solution d'extrait synthétique.

3. Extrait aqueux synthétique d'un organe selon les revendications 1 et 2, caractérisé par le fait que le groupe (I) comprend la glycine avec une quote-part de 1,5 à 4,5% en poids, de préférence de 2,4 à 3,6% en poids.

4. Extrait aqueux synthétique d'un organe selon les revendications 1 à 3, caractérisé par le fait que le composant (d) comprend le D-mannitol avec une quote-part de 3 à 7% en poids, de préférence de 4 à 6% en poids.

5. Extrait aqueux synthétique d'un organe selon les revendications 1 à 4, caractérisé par le fait qu'en tant que composant (g), au moins une quote-part de 15 à 50% en poids de D-panthénol, de préférence de 24 à 36% en poids est comprise dans la solution d'extrait synthétique.

6. Extrait aqueux synthétique d'un organe selon la revendication 5, caractérisé par le fait qu'il comprend de façon supplémentaire 0,8 à 1,2% en poids de monohydrate de D-lactose.

7. Extrait aqueux synthétique d'un organe selon la revendication 1, caractérisé par le fait que le composant aminoacidique (a) et le composant peptinique (b) comprennent environ

- 0,2 à 0,7% en poids d'aminoacides du groupe (I),
- 0,05 à 0,15% en poids d'aminoacides du groupe (II) et
- 0,10 à 0,20% en poids d'aminoacides du groupe (III)

et

- le composant (c) avec une quote-part de 0,02 à 0,06% en poids,
- le composant (d) avec une quote-part totale en sorbite, mannitol et inosite de 0,2 à 0,5% en poids,
- le composant (e) avec une quote-part de 0,2 à 0,7% en poids,
- le composant (f) avec une quote-part de 0,3 à 0,7% en poids,

respectivement par rapport à l'extrait total, étant compris dans la solution d'extrait synthétique.

8. Extrait aqueux synthétique d'un organe selon les revendications 1 à 7, caractérisé par le fait qu'il comprend de l'acide malique et/ou de l'acide succinique avec une quote-part de 0,02 à 1,5% en poids.

9. Extrait aqueux synthétique d'un organe selon les revendications 1 à 8, caractérisé par le fait que le composant (b) comprend des peptides qui contiennent au moins un des aminoacides choisis du groupe comprenant la sérine, l'arginine, la lysine, la proline et l'hydroxyproline.

10. Extrait aqueux synthétique d'un organe selon les revendications 1 à 9, caractérisé par le fait que le composant (b) comprend des peptides qui contiennent au moins deux des aminoacides sérine, arginine, lysine, proline, hydroxyproline et histidine.

11. Extrait aqueux synthétique d'un organe selon les revendications 1 à 10, caractérisé par le fait que le composant (b) comprend essentiellement des tri- à hexapeptides, leurs sels et/ou leurs complexes métalliques.

12. Extrait aqueux synthétique d'un organe selon les revendications 1 à 11, caractérisé par le fait que le composant (b) est composé au moins partiellement de peptones qui sont choisis du groupe consistant de peptone de soya, de peptone de mais, de peptone d'avoine, des peptides de levure et/ou de levure partiellement déshydratée.

13. Extrait aqueux synthétique d'un organe selon les revendications 1 à 12, caractérisé par le fait qu'il comprend des aminoacides monomères du groupe composé de glycine, de proline, de sérine, de lysine, d'arginine et d'hydroxyproline avec une quote-part de 0,05 à 5,0% en poids.

14. Extrait aqueux synthétique d'un organe selon les revendications 1 à 13, caractérisé par le fait qu'il comprend des hexites dans une quantité de 0,2 à 8% en poids.

15. Extrait aqueux synthétique d'un organe selon les revendications 1 à 14, caractérisé par le fait que le composant alcoolique (f) comprend le benzylalcool, la glycérine et/ou l'éthanol.

16. Extrait aqueux synthétique d'un organe selon les revendications 1 à 15, caractérisé par le fait que le composant (c) comprend l'inosine, l'adénine, l'adénosine, la guanosine, des AMP cycliques et/ou l'AMP.

17. Extrait aqueux synthétique d'un organe selon les revendications 1 à 16, caractérisé par le fait qu'il ne contient pas des agents conservateurs.

18. Extrait aqueux synthétique d'un organe selon les revendications 1 à 17, caractérisé par le fait que la teneur en matière sèche de la solution d'extrait synthétique d'un organe s'élève à 0,2 à 60% en poids.

19. Utilisation de l'extrait aqueux synthétique d'un organe selon les revendications 1 à 18 en tant que substitution ou complément pour des extraits de placenta, de thymus, de sang, de rate, de foie, de collagène, de tissu conjonctif, de liquide d'amnion, de méduse, d'oeufs de poisson et de pis, respectivement pour leurs combinaisons.

20. Utilisation de l'extrait aqueux synthétique d'un organe selon les revendications 1 à 18 pour la réalisation d'une formule cosmétique.

21. Utilisation d'une combinaison de deux ou plusieurs extraits aqueux synthétiques d'organes selon les revendications 1 à 18 pour la réalisation d'une formule cosmétique.

22. Utilisation de l'extrait aqueux synthétique d'un organe selon les revendications 1 à 18 pour la réalisation d'une préparation médicinale pour l'application topique, sous-cutanée ou intramusculaire aux fins de la cicatrisation externe ou interne.

23. Utilisation de l'extrait aqueux synthétique d'un organe selon les revendications 1 à 18 pour la réalisation d'une préparation médicinale pour la fortification du système immunitaire.

24. Utilisation de l'extrait aqueux synthétique d'un organe selon les revendications 1 à 18 pour la réalisation d'une préparation médicinale pour l'activation du métabolisme cellulaire.

25. Utilisation de l'extrait aqueux synthétique d'un organe selon les revendications 1 à 18 pour la réalisation d'une préparation médicinale pour le traitement de maladies gastro-énterologiques, en particulier des ulcères.

Figur 1: Gastro-Explantat unter dem Einfluß der gemäß
Beispiel 4 hergestellten synthetischen Organextraktlösung ohne Konservierungsmittel

Figur 2: Gastro-Explantat unter dem Einfluß der gemäß
Beispiel 4 hergestellten synthetischen Organextraktlösung mit Konservierungsmittel